# EUROPEAN PATENT APPLICATION

(11) **EP 3 950 727 A1**
(43) Date of publication of application: **09.02.2022**
(21) Application number: 20783016.7
(22) Date of filing: 12.03.2020
(51) Int. Cl.: C08F 6/10, A61Q 1/00, A61Q 15/00, A61Q 17/04, A61Q 19/00, C08F 220/18, C08F 230/08, C08K 5/05, C08L 43/04, C08L 91/00, C08F 2/00, A61K 8/31, A61K 8/34, A61K 8/891

(54) **COPOLYMER HAVING CARBOSILOXANE DENDRIMER STRUCTURE, AND COMPOSITION, COSMETIC INGREDIENT, COATING FORMING AGENT, AND COSMETIC CONTAINING SAME**

(30) Priority: 01.04.2019 JP 2019069813
(71) Applicant: Dow Toray Co., Ltd., Tokyo 140-8617 (JP)
(72) Inventor: SOUDA, Tatsuo, Ichihara-shi Chiba 299-0108 (JP); SUGIURA, Tsunehito, Ichihara-shi Chiba 299-0108 (JP); KENNOKI, Masakado, Ichihara-shi Chiba 299-0108 (JP); MATSUBA, Masashi, Ichihara-shi Chiba 299-0108 (JP)
(74) Representative: Murgitroyd & Company
(86) International application number: PCT/JP2020/010836
(87) International publication number: WO 2020/203145

(57) **Abstract**

Problem: To provide: a copolymer having a carbosiloxane dendrimer structure, where the amount of monomers having a carbosiloxane dendrimer structure is significantly reduced; a composition, cosmetic raw material, film-forming agent, and a cosmetic material containing the same; and a method of manufacturing the copolymer and the like.

Resolution Means: A copolymer of (a1) an unsaturated monomer having a carbosiloxane dendrimer structure having a radically polymerizable organic group, and (a2) an unsaturated monomer having a radically polymerizable vinyl group, which is different from component (a1), where the amount of unreacted unsaturated monomers (a1) and saturated monomers derived from the unreacted unsaturated monomers (a1) is 2500 ppm or less relative to the copolymer; and a use thereof.

## Description

### TECHNICAL FIELD

The present invention relates to a copolymer having a carbosiloxane dendrimer structure, where the amount of monomers having a carbosiloxane dendrimer structure is significantly reduced, and to a composition, cosmetic raw material, film-forming agent, and a cosmetic material containing the same, and further relates to a method of manufacturing the copolymer having a carbosiloxane dendrimer structure and a copolymer composition.

### BACKGROUND ART

It is conventionally known that a copolymer having a carbosiloxane dendrimer structure having excellent film-forming properties is used in cosmetic materials in order to improve the cosmetic retainability of the cosmetic material, water resistance, sebum resistance, and the like (for example, Patent Document 1 and the like). These copolymers having a carbosiloxane dendrimer structure are formed by a radical copolymerization reaction of an unsaturated monomer having a carbosiloxane dendrimer structure and another unsaturated monomer.

On the other hand, in recent years, in addition to improving the durability of cosmetic materials described above, there is demand for cosmetic materials that are hypoallergenic with regard to skin and hair. However, there is concern about the irritating properties of radically polymerizable unsaturated monomers, and there is a demand for a copolymer and copolymer composition with a small amount of unreacted monomers. However, unsaturated monomers having a carbosiloxane dendrimer structure or saturated monomers derived therefrom are monomers referred to as so-called macromonomers having a relatively high molecular weight, and have a problem of being generally difficult to remove from a copolymer or a composition thereof by distillation.

For example, Patent Document 1 described above includes a purifying method of re-precipitating the obtained copolymer with methanol, and then drying the obtained solid. However, a large amount of methanol was required, and it was difficult to re-disperse or dissolve the extracted solid in a cosmetic oil agent, which is not suitable for actual production of a liquid composition. Furthermore, the methanol used in the purification is irritative and toxic, and residue thereof is a concern. In addition, when a polar functional group such as a carboxylic acid modified group or the like is introduced to a copolymer having a carbosiloxane dendrimer structure, the molecule becomes polar, methanol becomes an inferior solvent, and it is necessary to change to another solvent. For example, with ethanol and the like, the obtained copolymer and the unsaturated monomer having a carbosiloxane dendrimer structure both dissolve, and therefore, problems occur where reprecipitation is not possible, and the like.

Furthermore, with a focus on reducing the odor of unsaturated monomers having a carbosiloxane dendrimer structure and other unsaturated monomers remaining in the system, Patent Document 2 proposes converting remaining saturated monomers into corresponding esters (saturated monomers) by a hydrogenation reaction to reduce odors. However, there is a problem where the same amount of hydrogenated unreacted monomers (saturated monomers) derived from the unsaturated monomer having the carbosiloxane dendrimer structure remains in the copolymer, and thus it is difficult to achieve the object of reducing monomers having a carbosiloxane dendrimer structure. Furthermore, there is a problem where the hydrogenation reaction itself requires a special device, and thus large scale production cannot be easily performed.

### Related Art Documents

### Patent Documents

Patent Document 1: Japanese Unexamined Patent Application 2000-63225
Patent Document 2: Japanese Unexamined Patent Application 2014-40512

### SUMMARY OF THE INVENTION

### Problem to be Solved by the Invention

In view of the foregoing problems and technical conditions, an object of the present invention is to provide: a copolymer having a carbosiloxane dendrimer structure having excellent hypoallergenicity and safety and having high purity by significantly reducing the amount of a monomer having a carbosiloxane dendrimer structure; and a composition, a cosmetic raw material, a film-forming agent, and a cosmetic material containing the copolymer. Furthermore, an object is to provide a method of manufacturing the copolymer having a carbosiloxane dendrimer structure and a copolymer composition, which can be easily implemented on an industrial production scale.

### Means for Solving the Problem

As a result of extensive studies, the present inventors discovered that the amount of monomers having a carbosiloxane dendrimer structure can be significantly reduced by introducing a polymerization initiator in two stages by providing a difference in introduction times in a radical copolymerization reaction between an unsaturated monomer having a carbosiloxane dendrimer structure and another unsaturated monomer, optionally adding another additional unsaturated monomer, advancing a reaction for the unreacted unsaturated monomer having a carbosiloxane dendrimer structure, and then after this step, distilling off unreacted unsaturated monomers or saturated monomers remaining in the system, thereby completing the present invention. The copolymer having a carbosiloxane dendrimer structure of the present invention is characterized in that the amount of monomers (unsaturated monomers and saturated monomers) having a carbosiloxane dendrimer structure, which are normally difficult to remove, is significantly lower.

In other words, a first aspect of the present invention is a copolymer of (a1) an unsaturated monomer having a carbosiloxane dendrimer structure having a radically polymerizable organic group, and (a2) an unsaturated monomer having a radically polymerizable vinyl group, which is different from component (a1), where the amount of unreacted unsaturated monomers (a1) and saturated monomers derived from the unreacted unsaturated monomers (a1) is 2500 ppm or less relative to the copolymer. Herein, the mass% of the aforementioned component (a1) is preferably 20 mass% or more relative to the total mass of components (a1) and (a2) configuring the copolymer.

A second aspect of the present invention is a copolymer composition containing the aforementioned copolymer and one or more types of (C) alcohols and/or (D) oil agents.

A third aspect of the present invention is a cosmetic raw material, film-forming agent, and cosmetic material containing the aforementioned copolymer or a composition thereof.

A fourth aspect of the present invention is a method of manufacturing the aforementioned copolymer, including:
a step of adding a polymerization initiator to (a1) the unsaturated monomer having a carbosiloxane dendrimer structure having a radically polymerizable organic group, and (a2) the unsaturated monomer having a radically polymerizable vinyl group, which is different from component (a1) to perform a radical polymerization reaction; and step (I): a step (Ia) of further adding the same or different polymerization initiator than the initially added polymerization initiator at least two hours after adding the initial polymerization initiator for the radical polymerization reaction.

The manufacturing method may optionally include:
a step (Ib) of adding a monomer (a2') having a radically polymerizable vinyl group with a boiling point less than 160°C at ambient pressure (1 atm) to advance the radical polymerization reaction of unreacted component (a1), in step (I); and step (II): a step of distilling off unreacted unsaturated monomers or saturated monomers remaining in the system at ambient pressure or under reduced pressure after step (I).

The manufacturing method may further include step (III): a step of performing a hydrogenation reaction on an unreacted unsaturated monomer remaining in the system to convert the unsaturated monomer into a saturated monomer, and in order to obtain the aforementioned copolymer composition, step (IV): a step of adding one or more types of (C) alcohols and/or (D) oil agents to the system to distill off a reaction solvent for a radical polymerization reaction.

### Effects of the Invention

The present invention can provide: a copolymer having a carbosiloxane dendrimer structure having excellent hypoallergenicity and safety and having high purity; and a composition, cosmetic raw material, film-forming agent, and cosmetic material containing the same. Furthermore, the present invention can provide a method of manufacturing the copolymer having a carbosiloxane dendrimer structure and a copolymer composition, which can be easily implemented on an industrial production scale.

### DESCRIPTION OF THE PREFERRED EMBODIMENT

In the present specification, "(meth)acrylic acid" indicates that both acrylic acid and methacrylic acid are included. Similarly, "(meth) acrylate", "(meth)acryloxy", and "(meth)acrylamide" also indicate that both acrylate and methacrylate, acryloxy and methacryloxy, and acrylamide and methacrylamide, respectively, are included. In the present specification, "cosmetic material" and "cosmetic product" are used interchangeably.

A copolymer having a carbosiloxane dendrimer structure of the present invention is obtained by copolymerizing: component (a1): an unsaturated monomer having a carbosiloxane dendrimer structure; and component (a2): an unsaturated monomer different from component (a1). Component (a1) is preferably an unsaturated monomer having a carbosiloxane dendrimer structure as expressed by Formula (1).

Formula (1): 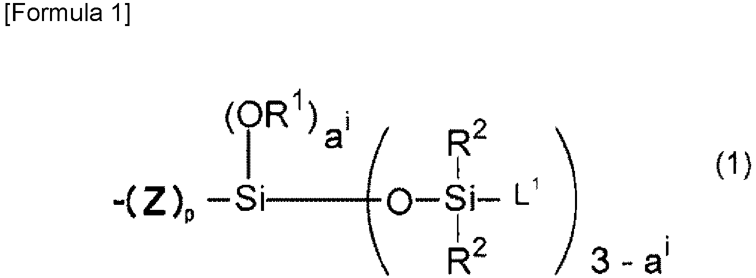 {where
Z represents a divalent organic group;
P represents 0 or 1;
R¹ and R² independently represent an alkyl group having 1 to 10 carbon atoms, an aryl group, or an aralkyl group; and
L¹ represents a silyl alkyl group as expressed by the following Formula (2) when i = 1 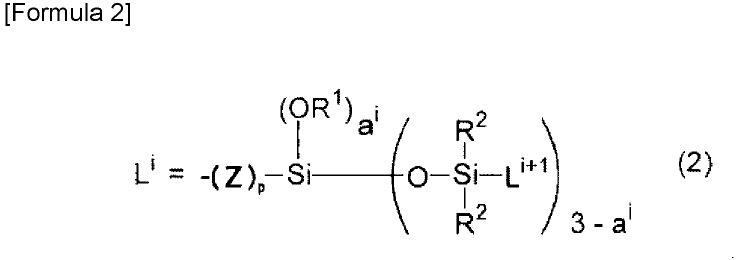
(where
Z and p are the same as described above;
R1 and R2 are the same as described above;
i represents an integer from 1 to 10 indicating the total hierarchical number of silyl alkyl groups; and
Lⁱ⁺¹ represents a group selected from a group consisting of a hydrogen atom, alkyl groups having 1 to 10 carbon atoms, aryl groups, aralkyl groups, and the silylalkyl groups; however, when i = c (c represents an integer from 1 to 10 indicating the hierarchy of the silylalkyl group), Lⁱ⁺¹ represents a hydrogen atom, an alkyl group having 1 to 10 carbon atoms, an aryl group, or an aralkyl group, but represents the silylalkyl group when i < c, and aⁱ represents an integer from 0 to 3)}.

Note that the carbosiloxane dendrimer structure is a chemical structure that is highly branched radially from a single silicon atom, and i, which indicates the total number of silyl alkyl groups in the hierarchy, indicates the degree of branching. For example, if the total number of hierarchies i is 1 and Lⁱ⁺¹ is, for example, a methyl group, then the carbosiloxane dendrimer structure refers to the following structure. 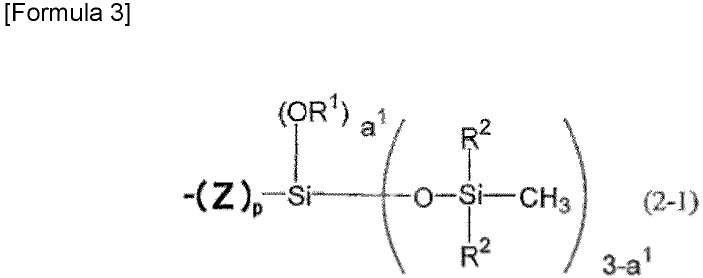 (where Z, p, R¹ and R² are the same as described above, and a¹ represents an integer between 0 and 3)

Similarly, if the hierarchy i is 2 and Lⁱ⁺¹ is, for example, a methyl group, then the carbosiloxane dendrimer structure refers to the following structure (however, p = 1). 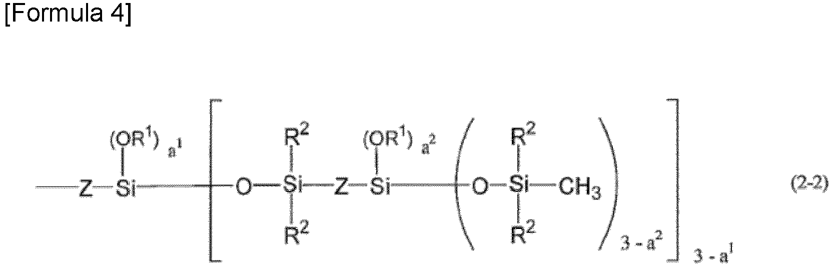 (where Z, R¹ and R² are the same as described above, and a¹ and a² represent an integer between 0 and 3)

Preferably, the aforementioned a, a¹, and a² represent 0, and the carbosiloxane dendrimer structure particularly preferably as follows. 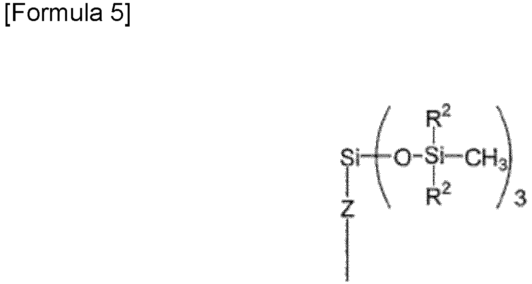 (Where Z and R² are the same as described above) 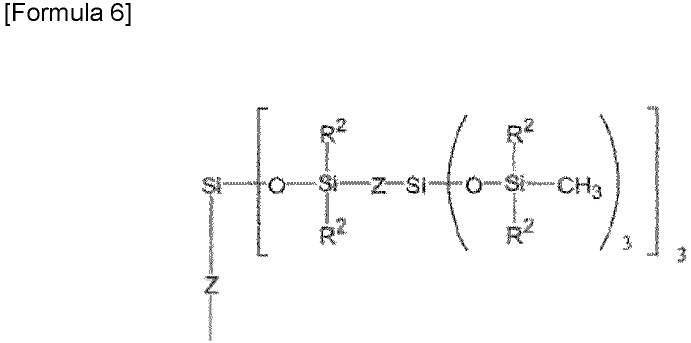 (Where Z and R² are the same as described above)

The unsaturated group of component (a1) is not limited so long as a radically polymerizable unsaturated group is provided, and examples include vinyl groups, allyl groups, (meth)acrylic groups, and the like. In particular, component (a1) is preferably an acrylic acid ester-based monomer or a methacrylic acid ester-based monomer having a carbosiloxane dendrimer structure described above. Of these, a particularly preferable unsaturated monomer is an unsaturated monomer where an unsaturated group as expressed by the following structures is bonded to a silicon atom (Si) directly or through - (Z) p- or -Z- in the carbosiloxane dendrimer structure described above.

An organic group containing an acrylic group or methacrylic group as expressed by 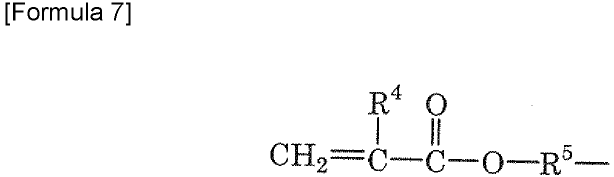 (Where R⁴ represents a hydrogen atom or a methyl group, and R⁵ represents an alkylene group having 1 to 10 carbon atoms.),
or 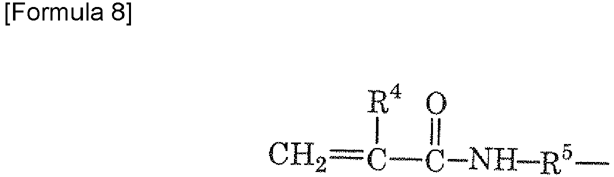 (Where R⁴ and R⁵ are the same as described above.).

A more specific example of this component includes an unsaturated monomer containing a carbosiloxane dendrimer structure as expressed by the following average composition formula.

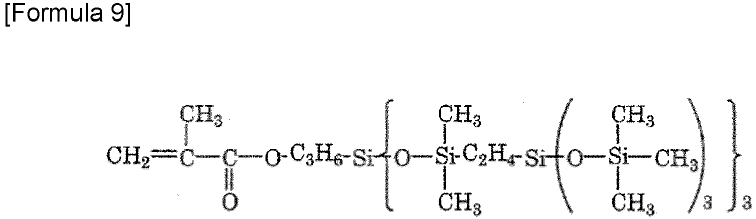

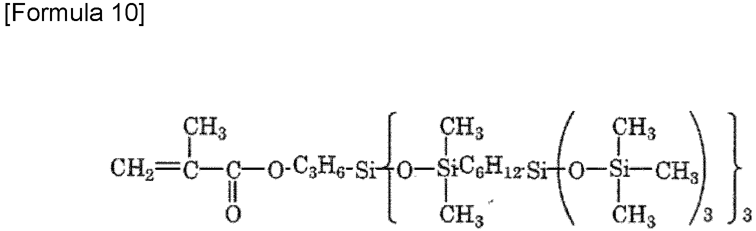

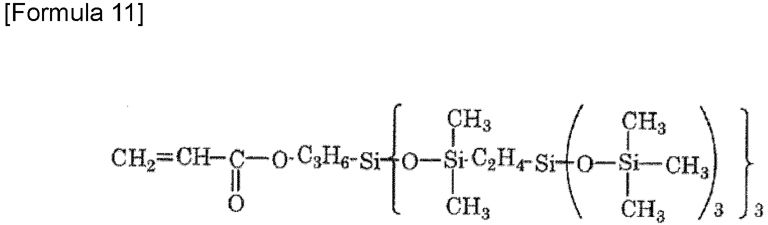

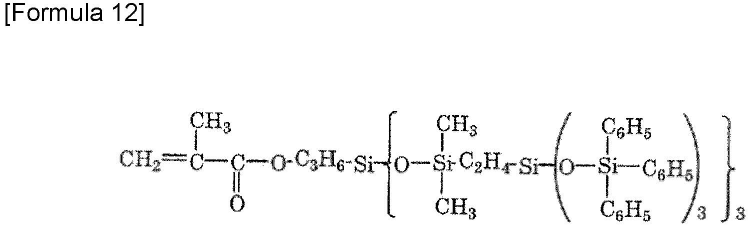

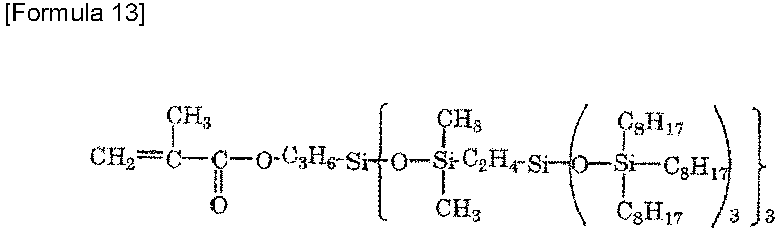

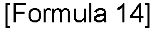

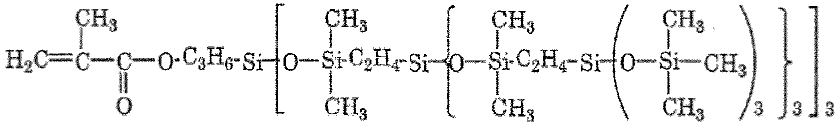

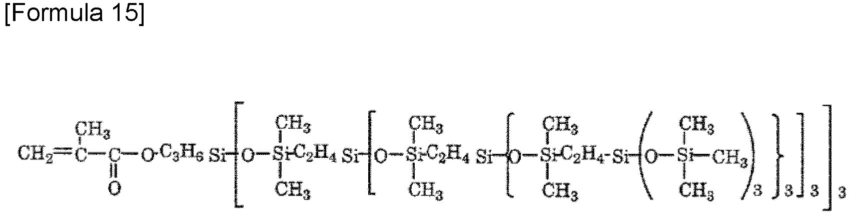

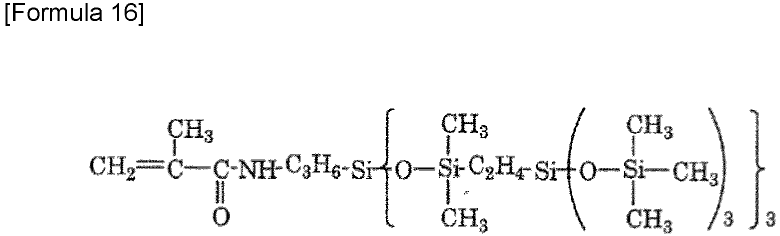

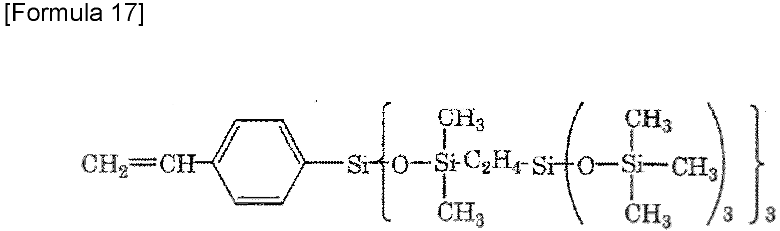

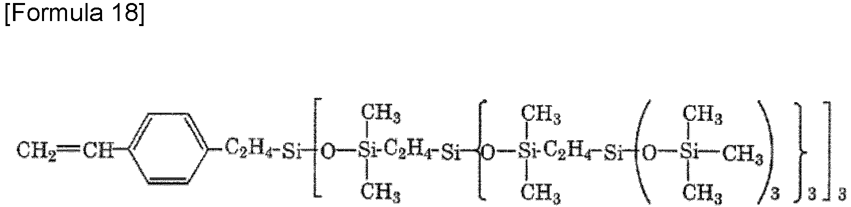

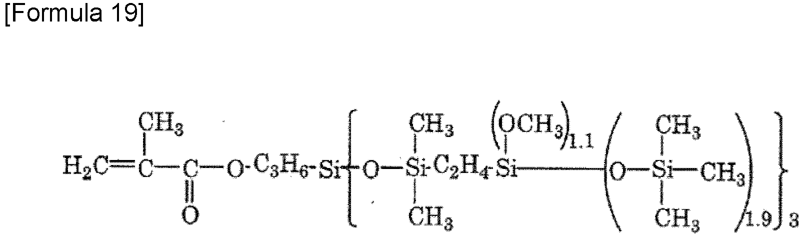

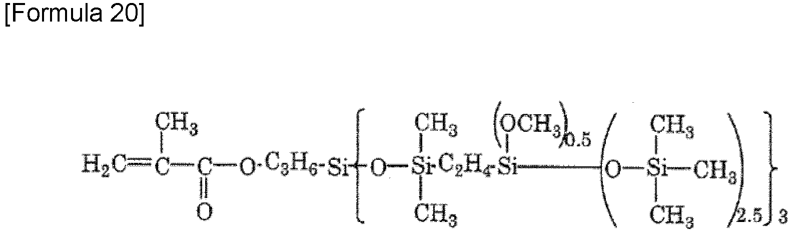

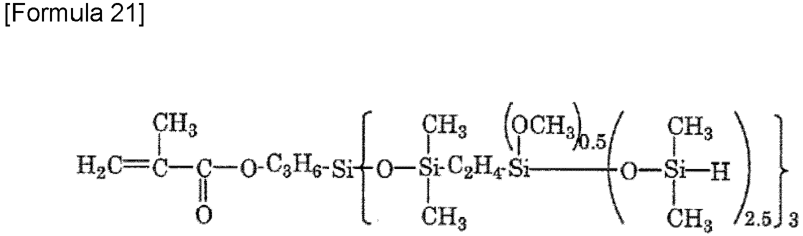

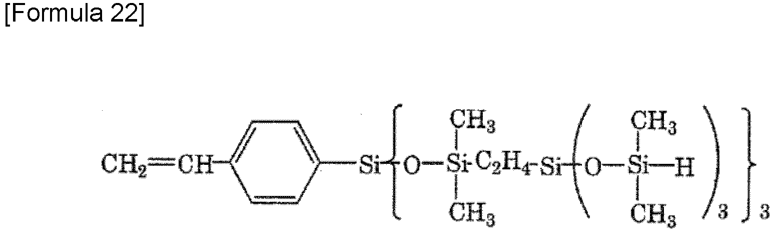

Polymerization of the unsaturated monomers having a carbosiloxane dendrimer structure can be manufactured in accordance with manufacturing methods disclosed in Japanese Unexamined Patent Application H11-1530, Japanese Unexamined Patent Application 2000-63225, Japanese Unexamined Patent Application 2001-192424, Japanese Unexamined Patent Application 2014-40512, and the like.

Component (A2) containing the copolymer having a carbosiloxane dendrimer structure of the present invention is an unsaturated monomer different from component (A1), and may be a monomer having a radical polymerizable vinyl group. The type and the like thereof is not particularly limited. More specifically, the vinyl-based monomer does not have a carbosiloxane dendrimer structure in a molecule.

Examples of the vinyl-based monomer include monomers that are starting materials of organic resins, which are generally called vinyl-based resins. Specific examples include: lower alkyl (meth)acrylates such as methyl (meth)acrylate, ethyl (meth)acrylate, n-propyl (meth)acrylate, and isopropyl (meth)acrylate; glycidyl (meth)acrylate; higher (meth)acrylates such as n-butyl (meth)acrylate, isobutyl (meth)acrylate, tert-butyl (meth)acrylate, n-hexyl (meth)acrylate, cyclohexyl (meth)acrylate, 2-ethylhexyl (meth)acrylate, octyl (meth)acrylate, lauryl (meth)acrylate, and stearyl (meth)acrylate; lower fatty acid vinyl esters such as vinyl acetate and vinyl propionate; higher fatty acid esters such as vinyl butyrate, vinyl caproate, vinyl 2-ethylhexanoate, vinyl laurate, and vinyl stearate; aromatic vinyl-based monomers such styrene, vinyl toluene, benzyl (meth)acrylate, phenoxyethyl (meth)acrylate, and vinyl pyrrolidone; vinyl-based monomers containing an amide group such as (meth)acrylamide, N-methylol (meth)acrylamide, N-methoxymethyl (meth)acrylamide, isobutoxymethoxy (meth)acrylamide, and N,N-dimethyl (meth)acrylamide; vinyl-type monomers containing a hydroxy group such as 2-hydroxyethyl (meth)acrylate, 2-hydroxybutyl (meth)acrylate, and 2-hydroxypropyl (meth)acrylate: fluorine-containing vinyl-type monomers such as trifluoropropyl (meth)acrylate, perfluorobutylethyl (meth)acrylate, and perfluorooctylethyl (meth)acrylate; vinyl-type monomers containing an epoxy group such as glycidyl (meth)acrylate and 3,4 epoxycyclohexylmethyl (meth)acrylate; carboxylic acid-containing vinyl-type monomers such as (meth)acrylic acid, itaconic acid, crotonic acid, fumaric acid, and maleic acid; ether bond-containing vinyl-based monomers such as tetrahydrofurfuryl (meth)acrylate, butoxyethyl (meth)acrylate, ethoxydiethylene glycol (meth)acrylate, polyethylene glycol (meth)acrylate, polypropylene glycol mono(meth)acrylate, hydroxybutyl vinyl ether, cetyl vinyl ether, and 2-ethylhexyl vinyl ether; silicone compounds containing an unsaturated group such as (meth)acryloxypropyl trimethoxysilane, (branched or linear) polydimethyl siloxane containing a (meth)acrylic group at one end, and a polydimethyl siloxane containing a styryl group at one end; butadiene; vinyl chloride; vinylidene chloride; (meth)acrylonitrile; dibutyl fumarate; maleic anhydride; dodecyl succinic anhydride; (meth)acrylic glycidyl ether; alkali metal salts, ammonium salts, organic amine salts of radically polymerizable unsaturated carboxylic acids such as (meth)acrylic acid, itaconic acid, crotonic acid, fumaric acid, and maleic acid; radical polymerizable unsaturated monomers having sulfonic acid groups such as styrene sulfonic acid, and their alkali metal salts, ammonium salts, organic amine salts; quaternary ammonium salts derived from (meth)acrylic acids, such as 2-hydroxy-3-methacryloxypropyl trimethylammonium chloride, methacrylates of alcohols with tertiary amine groups, such as diethylamine methacrylates, and quaternary ammonium salts thereof.

Furthermore, a polyfunctional vinyl-based monomer can be used, and examples thereof include trimethylolpropane tri(meth)acrylate, pentaerythritol tri(meth)acrylate, ethylene glycol di(meth)acrylate, tetraethylene glycol di(meth)acrylate, polyethylene glycol di(meth)acrylate, 1,4-butanediol di(meth)acrylate, 1,6-hexanediol di(meth)acrylate, neopentyl glycol di(meth)acrylate, trimethylolpropane trioxyethyl (meth)acrylate, tris(2-hydroxyethyl)isocyanurate di(meth)acrylate, tris(2-hydroxyethyl)isocyanurate tri(meth)acrylate, monomers containing a (meth)acryloyl group such as di(meth)acrylate of diol, ethylene oxide of bisphenol A or adduct of propylene oxide, di(meth)acrylate of diol, ethylene oxide of hydrogenated bisphenol A or adduct of propylene oxide, and triethylene glycol divinyl ether, silicone compounds containing an unsaturated group such as polydimethylsiloxanes blocked at both ends by a styryl group and polydimethylsiloxanes blocked at both ends by a methacryloxypropyl, and the like.

Furthermore, an organic silicon compound having a vinyl-based radical polymerizable unsaturated group and a hydrolyzable group can also be used. In this case, the film strength becomes hard and water repellency durability is improved, which is preferable. Here, examples of the radically polymerizable group include a (meth)acryloxy group-containing organic group, a (meth)acrylamide group-containing organic group, and a styryl group-containing organic group, as represented by the following general formulas, or an alkenyl group having from 2 to 10 carbon atoms, and a vinyloxy group and an allyloxy group.

Similarly, an unsaturated monomer having at least one acidic group or a salt thereof in a molecule can also be used. The unsaturated monomer having at least one acidic group or a salt thereof in a molecule is a compound having a radically polymerizable vinyl group and at least one acidic group or a salt thereof in a molecule. Examples of the acidic group include carboxylic acids, sulfonic acids, and phosphonic acids. Examples of salts thereof include alkali metal salts, alkaline earth metal salts, basic amino acid salts, ammonium salts, alkyl ammonium salts, alkyl amine salts, and alkanolamine salts, and specific examples include sodium salt, potassium salt, magnesium salt, calcium salt, L-arginine salt, L-histidine salt, L-lysine salt, ammonium salt, triethanolamine salt, aminomethyl propanediol salt, and complex salts thereof. Compounds having these acidic groups undergo a change in the hydrophilic-hydrophobic properties of the compound by releasing protons (H⁺) in an aqueous solution at respectively specific pH values or bonding with cationic components in the liquid to form a salt. Compounds with salts of acidic groups similarly undergo dissociation of the salt at a specific pH, and exhibit a change in the hydrophilic-hydrophobic properties of the compound. Therefore, by appropriately adding a compound having these acidic groups or salts thereof into a cosmetic material, an effect is achieved where washing away during cleaning is easy, even while also exhibiting favorable cosmetic retainability.

Similarly, for the purpose of improving water repellency or the like of the copolymer containing a carbosiloxane dendrimer structure in the present invention, an unsaturated monomer containing an organic group containing fluorine such as a perfluoroalkyl group and the like can also be used. An example is a vinyl-based monomer such as an acrylic monomer, a methacrylic monomer, or the like having an organic group containing fluorine such as a perfluoroalkyl group or the like.

The copolymer containing a carbosiloxane dendrimer structure in the present invention is obtained by the copolymerizing of the aforementioned component (a1) and component (a2), and the mass ratio at the time of the copolymerization is preferably within a range of (a1) : (a2) = 10:90 to 90:10, more preferably 20:80 to 85:15, and even more preferably 30:70 to 60:40. In particular, the mass% of the component (a1) described above is at least 20 mass%, and preferably at least 30 mass% relative to the total mass of component (a1) and component (a2), and component (a1) is particularly preferably 20 mass% to 60 mass% of total monomer units.

A step may be provided for the copolymer containing a carbosiloxane dendrimer structure according to the present invention in which a polymerization reaction is performed by adding a polymerization initiator to a raw material composition containing the monomer. Thereafter, a step may be provided in which the optionally obtained polymerization reaction product is contacted with a nickel catalyst or a palladium catalyst to perform a hydrogenation reaction.

As the polymerization method used in the polymerization reaction, a radical polymerization method or an ionic polymerization method is used, but a radical polymerization method is preferable. With regard to this radical polymerization method, a solution polymerization method is preferably used. The solution polymerization is performed by reacting the monomer composition containing component (a1) and component (a2) in a solvent at a temperature of 50 to 150°C for 3 to 20 hours in the presence of a radical initiator. Examples of the solvent used in the polymerization reaction include aliphatic hydrocarbons such as hexane, octane, decane, and cyclohexane; aromatic hydrocarbons such as benzene, toluene, and xylene; ethers such as diethyl ether, dibutyl ether, tetrahydrofuran, and dioxane; ketones such as acetone, methyl ethyl ketone, methyl isobutyl ketone, and diisobutyl ketone; esters such as methyl acetate, ethyl acetate, butyl acetate, and isobutyl acetate; alcohols such as methanol, ethanol, isopropyl alcohol, and butanol; and organosiloxane oligomers such as octamethylcyclotetrasiloxane, decamethylcyclopentasiloxane, hexamethyldisiloxane, and octamethyltrisiloxane. One of these solvents may be used alone, or two or more types thereof may be used in a mixture.

Conventionally known compounds commonly used in radical polymerization methods are used as the radical initiator, and specific examples include azobis-based compounds such as 2,2'-azobis(isobutyronitrile), 2,2'-azobis(2-methylbutyronitrile), and 2,2'-azobis(2,4-dimethylvaleronitrile); and organic peroxides such as benzoyl peroxide, lauroyl peroxide, tert-butylperoxy benzoate, tert-butylperoxy-2-ethylhexanoate, and tert-hexylperoxy-2-ethylhexanoate. One type of this radical initiator may be used alone, or two or more types may be mixed and used. The usage amount of the radical initiator is preferably in the range of from 0.1 to 5 parts by weight per a total of 100 parts by weight of the monomer composition.

In addition, a chain transfer agent can be added during polymerization. Specific examples of the chain transfer agent include mercapto compounds such as 2-mercaptoethanol, butyl mercaptan, n-dodecyl mercaptan, 3-mercaptopropyl trimethoxysilane, and polydimethylsiloxanes having a mercaptopropyl group; and halides such as methylene chloride, chloroform, carbon tetrachloride, butyl bromide, and 3-chloropropyl trimethoxysilane.

The polymerization reaction product can be contacted with a nickel catalyst or palladium catalyst, which are hydrogenation reaction catalysts. By being contacted with these catalysts, the vinyl groups of unreacted monomers remaining in the polymerization reaction product are saturated, and irritation and odor when added to the cosmetic material can be reduced. Nickel catalysts include, but are not necessarily limited to, nickel/diatomaceous earth and raney nickel catalysts. Examples of palladium catalysts include palladium compounds such as tetrakis (triphenylphosphine)palladium (0) and dichlorobis (triphenylphosphine)palladium (II); carbon-supported palladium, carbon-supported palladium hydroxide, and platinum oxide, but the palladium catalyst is not necessarily limited to these.

The temperature when contacting the polymerization reaction product with the nickel catalyst or palladium catalyst is 50 to 200°C, and preferably 70 to 130°C. The pressure is from 1 to 1000 kg/cm² (absolute pressure), and preferably from 2 to 100 kg/cm². The contact time is from 1 to 15 hours, and preferably from 3 to 10 hours. The reaction can be performed in a solvent, and the solvent that is used during polymerization may be used as is, or the solvent may be substituted. The solvents that can be used are the same as those described for the polymerization reaction.

[Amount of Monomer Having Carbosiloxane Dendrimer Structure and Significance Thereof]

A copolymer containing a carbosiloxane dendrimer structure according to the present invention is characterized in that the amount of the unreacted unsaturated monomer (a1) and the saturated monomer derived from the unreacted unsaturated monomer (a1) is 2500 ppm or less with regard to the copolymer. Preferably, the amount of the monomers having a carbosiloxane dendrimer structure is 2,400 ppm or less. When the manufacturing method described below is used, a copolymer with an amount reduced to 2,000 ppm or less, or 1,500 ppm or less, can also be manufactured as necessary. Thus, a copolymer having an extremely low amount of monomers containing a carbosiloxane dendrimer structure can be obtained as a cosmetic raw material.

An object of the present invention is to provide a copolymer with a reduced amount of monomers containing a carbosiloxane dendrimer structure (unsaturated monomers and saturated monomers which are hydrogenated products thereof) having properties which are of concern for irritation and which are difficult to separate or remove from the copolymer by ordinary distilling means. However, when the amount of unreacted unsaturated monomer (a1) and saturated monomer derived from the unreacted unsaturated monomers (a1) is reduced to less than the aforementioned reduction, the amount of the copolymer used in a general composition as a cosmetic raw material or a film-forming agent is approximately 4 mass% of the entire cosmetic material, and when it is arranged in a cosmetic material (including a topical agent and a quasi-pharmaceutical product), the amount of the monomer in the final composition or formulation is 100 ppm or less. Thus the effects thereof are practically negligible level. Note that the amount of the monomer in the copolymer can be reduced by a method described below, and even if the amount of the monomer is reduced, the function of the copolymer containing a carbosiloxane dendrimer structure as a cosmetic raw material or film-forming agent is not impaired.

The amount of the unreacted unsaturated monomer (a1) and the saturated monomer derived from the unreacted unsaturated monomer (a1) in the copolymer described above can be confirmed by means such as high performance liquid chromatography (HPLC) or the like, and can be calculated based on the peak intensity thereof. Furthermore, even for a copolymer composition, cosmetic raw material, or film-forming agent in which the copolymer is dispersed in an (D) organic solvent or the like, the amount of the monomer in a copolymer portion, excluding the copolymer alone or dispersant thereof, can be easily calculated. Furthermore, the amount of the unreacted unsaturated monomer (a1) and the saturated monomer derived from the unreacted unsaturated monomer (a1) in the final cosmetic material can also be specified with relatively high precision by a similar method, and the cosmetic material containing the copolymer according to the present invention in which the amount of the monomer is 100 ppm or less relative to the entire cosmetic material is included in preferred aspect of the present invention.

For example, when the copolymer in which the aforementioned specified unreacted monomer is reduced and is blended in the cosmetic material, the blending amount thereof is not particularly limited, but blending is possible within a range of 0.1 to 4 mass%, and preferably 1 to 4 weight% as the mass of the copolymer of the present invention alone, not including a solvent or the like. In particular, when blended in the range, it is possible to provide a cosmetic material which can impart functions such as film-forming properties and the like to the cosmetic material, which contains 100 ppm or less of a monomer having a carbosiloxane dendrimer structure, having properties which are of concern for irritation and which is difficult to separate or remove from the copolymer by ordinary distilling means, and which does not essentially contain the monomer.

Similarly, for a copolymer composition, cosmetic raw material, or film-forming agent in which the copolymer where the aforementioned specified unreacted monomer is reduced and dispersed in an (D) organic solvent and the like described below, and in which the amount of the copolymer is 40 mass%, it is possible to provide a copolymer composition, cosmetic raw material, or film-forming agent which can impart functions such as film-forming properties and the like to the cosmetic material, which contains 1000 ppm or less of a monomer having a carbosiloxane dendrimer structure, having properties which are of concern for irritation and which is difficult to separate or remove from the copolymer by ordinary distilling means, and which does not essentially contain the monomer.

The copolymer of the present invention can be blended into a cosmetic material or the like as is, or in the form of a composition such as being dissolved in a solvent, dispersed in a dispersing medium, or the like. Although these solvents and dispersants are not particularly limited, examples include (C) alcohols and (D) oil agents. In particular, a form of a copolymer composition containing a dispersant selected from one or more types of (C) alcohols and (D) oil agents is particularly preferred for practical use as a cosmetic raw material or film-forming agent.

### [Copolymer Composition]

In other words, the copolymer composition of the present invention is a composition containing a copolymer in which the specific unreacted monomer described above is reduced and one or more types selected from (C) alcohols and (D) oil agents is contained. When the copolymer, which is the first invention of the present invention, is compounded into a cosmetic material, the resulting form can be a solution with the copolymer dissolved in a solvent, a dispersion liquid with the copolymer dispersed in a dispersion medium, or a solid such as a powder, granules, or blocks. In particular, the copolymer according to the present invention is preferably dissolved or dispersed in one or more types of (C) alcohols or (D) oil agents and compounded in a cosmetic material in the form of a copolymer composition containing the copolymer and these dispersing media.

The copolymer of the present invention excels in miscibility with and dispersibility in various oil agents, and a polymer composition that remains homogeneous over an extended period of time can be obtained. Such a composition can be added as is into a cosmetic material, and is extremely useful as a cosmetic raw material or film-forming agent from the perspective of handling properties and storage stability thereof. Specifically, a copolymer composition can be preferably used, which contains 5 to 1000 parts by mass, preferably 50 to 500 parts by mass, and more preferably 100 to 400 parts by mass of at least one type of oil agent, with regard to 100 parts by mass of the copolymer of the present invention. Note that when a copolymer composition containing a copolymer and an oil agent is obtained, a copolymer from which a solvent and unreacted monomers are removed after the polymerization reaction may be homogeneously dispersed in an oil agent using mechanical force, and the volatile solvent during the polymerization reaction may be substituted with the abovementioned oil agent, which is particularly preferable. Note that as described above, for a copolymer composition containing (C) alcohols and/or (D) oil agents as dispersing media and having a copolymer amount of 40 mass%, it is possible to provide or design a composition in which the monomer containing a carbosiloxane dendrimer structure is 1000 ppm or less.

### (C) Alcohols

The copolymer according to the present invention may be used by dispersing or dissolving in an alcohol, and a specified affinity with alcohols, which are a general purpose component of a cosmetic material, and therefore, the copolymer of the present invention can also be coexisting as a compounding formulation for a cosmetic material. As the alcohols, one or more polyhydric alcohols and/or lower monohydric alcohols can be used. Examples of the lower alcohols include ethanol, isopropanol, n-propanol, t-butanol, and sec-butanol, and ethanol is preferable. Examples of polyhydric alcohols include dihydric alcohols such as 1,3-propanediol, 1,3-butylene glycol, 1,2-butylene glycol, propylene glycol, trimethylene glycol, tetramethylene glycol, 2,3-butylene glycol, pentamethylene glycol, 2-butene-1,4-diol, dibutylene glycol, pentyl glycol, hexylene glycol, and octylene glycol; trihydric alcohols such as glycerin, trimethylolpropane, and 1,2,6-hexanetriol; tetravalent or higher polyhydric alcohols such as pentaerythritol and xylitol; and sugar alcohols such as sorbitol, mannitol, maltitol, maltotriose, sucrose, erythritol, glucose, fructose, starch degradation products, maltose, xylitose, and starch-degrading sugarreducing alcohols. Other examples in addition to these low molecular weight polyhydric alcohols include polyhydric alcohol polymers such as diethylene glycol, dipropylene glycol, triethylene glycol, polypropylene glycol, tetraethylene glycol, diglycerin, polyethylene glycol, triglycerin, tetraglycerin, and polyglycerin. Of these, ethanol, 1,3-propanediol, 1,3-butylene glycol, sorbitol, dipropylene glycol, glycerin, and polyethylene glycol are particularly preferable. Of these alcohols, isopropanol and the like can also be used as the aforementioned reaction solvent, and therefore, a polymerization reaction composition containing these alcohols may be used as is as a copolymer composition.

### (D) Oil Agent

The copolymer of the present invention may be used by dispersing or dissolving in an oil agent, and examples of the oil agents described above includes animal oils, vegetable oils, synthetic oils, and the like, which are generally used for cosmetic materials. The oil agent may be a solid, semi-solid, or liquid, and may be non-volatile, semi-volatile, or volatile. The oil agent is used to impart lubricity to the skin or hair, and to soften the skin and impart a moisturized feeling. The oil agent can also be used to dilute the copolymer of the present invention and obtain a copolymer composition, and is particularly a liquid at 5 to 100°C. The oil agent is preferably at least one type selected from a silicone oil agent (D1) and an organic oil agent (D2), and the type and viscosity, etc. of these oil agents can be appropriately selected according to the type of cosmetic material and the application. These oil agents are compounded in the cosmetic material of the present invention at the same time as the copolymer composition.

### (D1) Silicone-Based Oil Agent

Silicone-based oil agents are generally hydrophobic, and their molecular structure may be linear, cyclic, or branched. The viscosities of silicone oils at 25°C are usually in the range of from 0.65 to 100,000 mm²/s, and preferably in the range of from 0.65 to 10,000 mm²/s. Furthermore, the silicone-based oil agent may exhibit volatility, and is preferably volatile.

Examples of silicone oils include cyclic organopolysiloxanes, linear organopolysiloxanes, and branched organopolysiloxanes. Among these, linear organopolysiloxanes, branched organopolysiloxanes, and cyclic organopolysiloxanes that are volatile are preferable.

As the silicone oil, for example, organopolysiloxanes expressed by the following general formulas (3), (4), or (5) can be used. 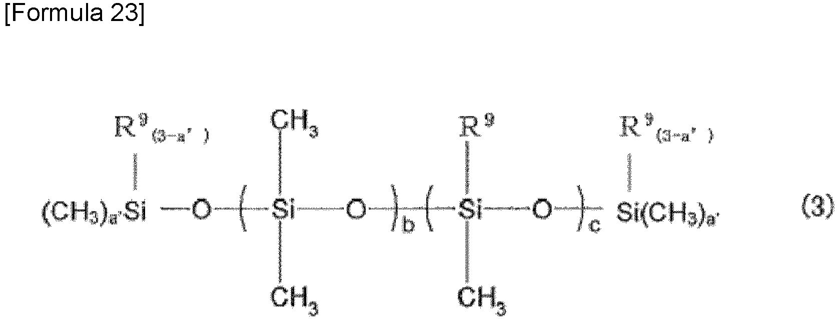 (Wherein, R⁹ is a hydrogen atom, a hydroxyl group, or a group selected from a monovalent unsubstituted or fluorine- or amino-substituted alkyl group, aryl group, and alkoxy group, having from 1 to 30 carbon atoms, and a group expressed by (CH₃)₃SiO{(CH₃)₂SiO}ₗSi(CH₃)₂CH₂CH₂- (where I is an integer from 0 to 1000); a' is an integer from 0 to 3; b is an integer from 0 to 1000; and c is an integer from 0 to 1000, provided that 1 ≦ b + c ≦ 2000.) 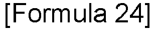 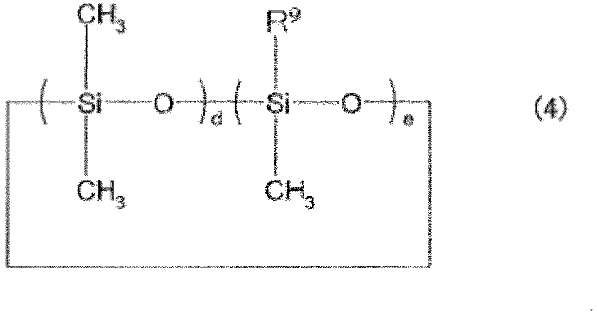 (Wherein, R⁹ is the same as above, d is an integer from 0 to 8, and e is an integer from 0 to 8, provided that 3 ≦ d + e ≦ 8.) 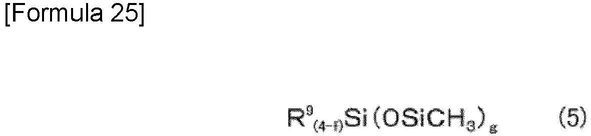 (Wherein, R⁹ is the same as described above, f is an integer from 1 to 4, and g is an integer from 0 to 500.)

Examples of the monovalent unsubstituted or fluorine- or amino-substituted alkyl group, aryl group, and alkoxy group having from 1 to 30 carbon atoms include a methyl group, ethyl group, propyl group, butyl group, pentyl group, hexyl group, heptyl group, octyl group, decyl group, dodecyl group, and other such linear or branched alkyl groups having from 1 to 30 carbon atoms; cycloalkyl groups having from 3 to 30 carbon atoms such as cyclopentyl groups and cyclohexyl groups; aryl groups having from 6 to 30 carbon atoms such as phenyl groups, tolyl groups, xylyl groups, and naphthyl groups; alkoxy groups having from 1 to 30 carbon atoms such as methoxy groups, ethoxy groups, and propoxy groups; and groups in which hydrogen atoms bonded to carbon atoms of these groups are at least partially substituted with fluorine or an amino group. An unsubstituted alkyl group or aryl group is preferable, an unsubstituted alkyl group or aryl group having from 1 to 6 carbon atoms is more preferable, and a methyl group, ethyl group, or phenyl group is particularly preferable.

More specific examples of silicone oils having these structures include, as cyclic organopolysiloxanes, hexamethylcyclotrisiloxane, octamethylcyclotetrasiloxane, decamethylcyclopentasiloxane, dodecamethylcyclohexasiloxane, 1,1-diethylhexamethylcyclotetrasiloxane, phenylheptamethylcyclotetrasiloxane, 1,1-diphenylhexamethylcyclotetrasiloxane, 1,3,5,7-tetravinyltetramethylcyclotetrasiloxane, 1,3,5,7-tetramethylcyclotetrasiloxane, 1,3,5,7-tetracyclohexyltetramethylcyclotetrasiloxane, tris(3,3,3-trifluoropropyl) trimethylcyclotrisiloxane, 1,3,5,7-tetra(3-methacryloxypropyl)tetramethylcyclotetrasiloxane, 1,3,5,7-tetra(3-acryloxypropyl)tetramethylcyclotetrasiloxane, 1,3,5,7-tetra(3-carboxypropyl)tetramethylcyclotetrasiloxane, 1,3,5,7-tetra(3-vinyloxypropyl)tetramethylcyclotetrasiloxane, 1,3,5,7-tetra(p-vinylphenyl)tetramethylcyclotetrasiloxane, 1,3,5,7-tetra[3-(p-vinylphenyl)propyl]tetramethylcyclotetrasiloxane, 1,3,5,7-tetra(N-acryloyl-N-methyl-3-aminopropyl)tetramethylcyclotetrasiloxane, and 1,3,5,7-tetra(N,N-bis(lauroyl)-3-aminopropyl) tetramethylcyclotetrasiloxane.

Examples of linear organopolysiloxanes include dimethylpolysiloxane capped at both molecular chain ends with trimethylsiloxy groups (dimethylsilicone ranging from a low viscosity such as 2 mPa·s or 6 mPa·s to a high viscosity such as 1,000,000 mPa·s), organohydrogenpolysiloxane, methylphenylpolysiloxane capped at both molecular chain ends with trimethylsiloxy groups, dimethylsiloxane/methylphenylsiloxane copolymer capped at both molecular chain ends with trimethylsiloxy groups, diphenylpolysiloxane capped at both molecular chain ends with trimethylsiloxy groups, dimethylsiloxane/diphenylsiloxane copolymer capped at both molecular chain ends with trimethylsiloxy groups, trimethylpentaphenyl trisiloxane, phenyl (trimethylsiloxy) siloxane, methyl alkyl polysiloxane capped at both molecular chain ends with trimethylsiloxy groups, dimethylpolysiloxane/methylalkylsiloxane copolymer capped at both molecular chain ends with trimethylsiloxy groups, dimethylsiloxane/methyl (3,3,3-trifluoropropyl) siloxane capped at both molecular chain ends with trimethylsiloxy groups, α,ω-dihydroxypolydimethylsiloxane, α,ω-diethoxypolymethylsiloxane, 1,1,1,3,5,5,5-heptamethyl-3-octyltrisiloxane, 1,1,1,3,5,5,5-heptamethyl-3-dodecyltrisiloxane, 1,1,1,3,5,5,5-heptamethyl-3-hexadecyltrisiloxane, tristrimethylsiloxymethylsilane, tristrimethylsiloxyalkylsilane, tetrakistrimethylsiloxysilane, tetramethyl-1,3-dihydroxydisiloxane, octamethyl-1,7-dihydroxytetrasiloxane, hexamethyl-1,5-diethoxytrisiloxane, hexamethyldisiloxane, octamethyltrisiloxane, higher alkoxy modified silicones, and higher fatty acid modified silicones.

Examples of the branched organopolysiloxane include methyltristrimethylsiloxysilane, ethyltristrimethylsiloxysilane, propyltristrimethylsiloxysilane, tetrakistrimethylsiloxysilane, and phenyltristrimethylsiloxysilane.

For a case in which the cosmetic material or the composition of the present invention is used as a cosmetic raw material, when at least one type of these silicone-based oil agents is included, the stability over time can be improved, and a refreshing tactile sensation that is unique to silicone oil can be achieved. Particularly preferably, of the silicone-based oil agents described above, decamethylcyclopentasiloxane, 1,1,1,3,5,5,5-heptamethyl-3-octyltrisiloxane (also referred to as "caprylyl methicone"), which is a linear organopolysiloxane having a viscosity in a low viscosity range of from 2 to 6 mPa·s, and tristrimethylsiloxymethylsilane (also referred to as "M3T"), etc. are used.

### (D2) Organic-Based Oil Agent

The organic-based oil agent is exemplified by (D2-1) hydrocarbon oils, (D2-2) fatty acid ester oils, higher alcohols, higher fatty acids, fats, and fluorine-based oil agents, and in the present invention, the organic-based oil agent is not particularly limited, but is preferably a liquid at temperatures from 5 to 100°C. Furthermore, hydrocarbon oils and/or fatty acid ester oils are preferred. These may be used alone or in combination, and can be used in combination with the silicone-based oil agent. By combining appropriate oil agents, the stability over time of the composition and/or cosmetic material can be improved, and a tactile sensation required for each cosmetic material can be imparted. A refreshing tactile sensation that is unique to silicone oil can be imparted by blending in the silicone-based oil agent, and a tactile sensation that is refreshing on the skin can be imparted by using an oil agent with high volatility. Furthermore, a moisturized sensation (also referred to as a "moisturized feel") and a smooth tactile sensation like that of damp skin or hair can be imparted by using a hydrocarbon oil and/or a fatty acid ester oil in combination with the silicone-based oil agent.

Examples of (D2-1) hydrocarbon oils include liquid paraffin, light liquid isoparaffin, heavy liquid isoparaffin, Vaseline, n-paraffin, isoparaffin, isododecane, isohexadecane, polyisobutylene, hydrogenated polyisobutylene, polybutene, ozokerite, ceresin, microcrystalline wax, paraffin wax, polyethylene wax, polyethylene polypropylene wax, squalane, squalene, pristane, and polyisoprene. In particular, in the composition according to the present invention, the use of isododecane is preferable because isododecane excels in volatility, excels in miscibility and affinity (compounding stability) with other cosmetic raw materials, and can impart a refreshing tactile sensation on the skin.

Examples of (D2-2) fatty acid ester oils include hexyldecyl octanoate, cetyl octanoate, isopropyl myristate, isopropyl palmitate, butyl stearate, hexyl laurate, myristyl myristate, oleyl oleate, decyl oleate, octyldodecyl myristate, hexyldecyl dimethyl octanoate cetyl lactate, myristyl lactate, diethyl phthalate, dibutyl phthalate, lanolin acetate, ethylene glycol monostearate, propylene glycol monostearate, propylene glycol dioleate, glyceryl monostearate, glyceryl monooleate, glyceryl tri-2-ethylhexanoate, trimethylolpropane tri-2-ethylhexanoate, ditrimethylolpropane triethylhexanoate, ditrimethylolpropane (isostearate/sebacate), trimethylolpropane trioctanoate, trimethylolpropane triisostearate, diisopropyl adipate, diisobutyl adipate, 2-hexyldecyl adipate, di-2-heptyl undecyl adipate, diisostearyl malate, hydrogenated castor oil monoisostearate, N-alkyl glycol monoisostearate, octyldodecyl isostearate, isopropyl isostearate, isocetyl isostearate, ethylene glycol di-2-ethylhexanoate, cetyl 2-ethylhexanoate, pentaerythritol tetra-2-ethylhexanoate, octyldodecyl gum esters, ethyl oleate, octyldodecyl oleate, neopentyl glycol dicaprate, triethyl citrate, 2-ethylhexyl succinate, dioctyl succinate, isocetyl stearate, diisopropyl sebacate, di-2-ethylhexyl sebacate, diethyl sebacate, dioctyl sebacate, dibutyl octyl sebacate, cetyl palmitate, octyldodecyl palmitate, octyl palmitate, 2-ethylhexyl palmitate, 2-hexyldecyl palmitate, 2-heptyl undecyl palmitate, cholesteryl 12-hydroxystearylate, dipentaerythritol fatty acid esters, 2-hexyldecyl myristate, ethyl laurate, 2-octyldodecyl N-lauroyl-L-glutamate, di-(cholesteryl/behenyl/octyldodecyl) N-lauroyl-L-glutamate, di-(cholesteryl/octyldodecyl) N-lauroyl-L-glutamate, di-(phytosteryl/behenyl/octyldodecyl) N-lauroyl-L-glutamate, di-(phytosteryl/octyldodecyl) N-lauroyl-L-glutamate, N-lauroyl sarcosine isopropyl, diisostearyl malate, neopentyl glycol dioctanoate, isodecyl neopentanoate, isotridecyl neopentanoate, isostearyl neopentanoate, isononyl isononanoate, isotridecyl isonononanoate, octyl isonononanoate, isotridecyl isonononanoate, diethyl pentanediol dineopentanoate, methyl pentanediol dineopentanoate, octyldodecyl neodecanoate, 2-butyl-2-ethyl-1,3-propanediol dioctanoate, pentaerythrityl tetraoctanoate, hydrogenated rosin pentaerythrityl, pentaerythrityl triethylhexanoate, dipentaerythrityl (hydroxystearate/stearate/rosinate), polyglyceryl tetraisostearate, polyglyceryl nonaisostearate-10, polyglyceryl deca(erucate/isostearate/lysinoleate)-8, diglyceryl oligoester (hexyl decanoate/sebacate), glycol distearate (ethylene glycol distearate), diisopropyl dimer linoleate, diisostearyl dimer linoleate, di-(isostearyl/phytostearyl) dimer dilinoleate, (phytostearyl/behenyl) dimer dilinoleate, (phytostearyl/isostearyl/cetyl/stearyl/behenyl) dimer dilinoleate, dimer dilinoleyl dimer dilinoleate, dimer dilinoleyl diisostearate, dimer dilinoleyl hydrogenated rosin condensate, hardened castor oil dimer dilinoleate, hydoxyalkyl dimer dilinoleyl ethers, glyceryl triisostearate, glyceryl trimyristate, glyceryl triisopalmitate, glyceryl trioctanoate, glyceryl trioleate, glyceryl diisostearate, glyceryl tri-(caprylate/caprate), glyceryl tri-(caprylate/caprate/myristate/stearate), glyceryl hydrogenated rosin glyceryl, hydrogenated rosin triglyceride (hydrogenated ester gum), rosin triglyceride (ester gum), glyceryl eicosanedioate behenate, glyceryl di-2-heptyl undecanoate, diglyceryl isostearate myristate, cholesteryl acetate, cholesteryl nonanoate, cholesteryl stearate, cholesteryl isostearate, cholesteryl oleate, cholesteryl 12-hydroxystearate, macadamia nut oil fatty acid cholesteryl, macadamia nut oil fatty acid phytosteryl, phytosteryl isostearate, soft lanolin fatty acid cholesteryl, hard lanolin fatty acid cholesteryl, long-chain branched fatty acid cholesteryl, long-chain α-hydroxy fatty acid cholesteryl, octyldodecyl ricinoleate, lanolin fatty acid octyldodecyl, octyldodecyl erucate, hardened castor oil isostearate, avocado oil fatty acid ethyl, lanolin fatty acid isopropyl, and the like. Lanolin and lanolin derivatives can also be used as fatty acid ester oils.

In addition to the above, oils and fats, higher alcohols, higher fatty acids, and fluorine-based oils, etc. may be used as oil agents, and two or more of these may be used in combination. For example, two or more types of oil agents represented below may be used in combination. More specific examples of other oil agents that can be used in the present invention are provided below. More specifically, use of one or more types selected from oils and fats, higher alcohols, higher fatty acids, and fluorine-based oil agents is exemplified.

Examples of oils and fats include, as natural animal and vegetable oils and fats and semi-synthetic oils and fats, avocado oil, linseed oil, almond oil, ibota wax, perilla oil, olive oil, cacao butter, kapok wax, kaya oil, carnauba wax, liver oil, candelilla wax, beef tallow, neatsfoot oil, beef bone fat, hydrogenated beef tallow, apricot kernel oil, spermaceti wax, hydrogenated oil, wheat germ oil, sesame oil, rice germ oil, rice bran oil, sugar cane wax, sasanqua oil, safflower oil, shea butter, Chinese tung oil, cinnamon oil, jojoba wax, olive squalane, shellac wax, turtle oil, soybean oil, tea seed oil, camellia oil, evening primrose oil, corn oil, pork fat, rapeseed oil, Japanese tung oil, rice bran wax, germ oil, horse fat, persic oil, palm oil, palm kernel oil, castor oil, hydrogenated castor oil, fatty acid methyl ester of castor oil, sunflower oil, grape oil, bayberry wax, jojoba oil, hydrogenated jojoba ester, macadamia nut oil, beeswax, mink oil, cottonseed oil, cotton wax, Japanese wax, Japanese wax kernel oil, montan wax, coconut oil, hydrogenated coconut oil, tricoconut oil fatty acid glyceride, mutton tallow, groundnut oil, lanolin, liquid lanolin, reduced lanolin, lanolin alcohol, hard lanolin, lanolin acetate, isopropyl lanolin fatty acid, POE lanolin alcohol ether, POE lanolin alcohol acetate, polyethylene glycol lanolin fatty acid, POE hydrogenated lanolin alcohol ether, and egg yolk oil. POE of course means polyoxyethylene.

Examples of higher alcohols include higher alcohols having from 10 to 30 carbon atoms. The higher alcohol is a saturated or unsaturated monohydric aliphatic alcohol, and the hydrocarbon group portion may be either linear or branched, but is more preferably linear. Examples of higher alcohols having from 10 to 30 carbon atoms include lauryl alcohol, myristyl alcohol, palmityl alcohol, stearyl alcohol, behenyl alcohol, hexadecyl alcohol, oleyl alcohol, isostearyl alcohol, hexyldodecanol, octyldodecanol, cetostearyl alcohol, 2-decyltetradecinol, cholesterol, sitosterol, phytosterol, lanosterol, lanolin alcohol, hydrogenated lanolin alcohol, POE cholesterol ether, monostearyl glycerin ether (batyl alcohol), and monooleyl glyceryl ether (selachyl alcohol). In the present invention, it is preferable to use a higher alcohol having a melting point of from 40 to 80°C alone, or to combine a plurality of higher alcohols so that the melting point is from 40 to 70°C. Such higher alcohols, together with surfactants, form assemblies called alpha gels and act to thicken the viscosity of the formulation and stabilize the emulsion, and therefore are particularly useful as a base agent for cosmetic materials in emulsion form.

Examples of higher fatty acids include lauric acid, myristic acid, palmitic acid, stearic acid, behenic acid, undecylenic acid, oleic acid, linoleic acid, linolenic acid, arachidonic acid, eicosapentaenoic acid (EPA), docosahexaenoic acid (DHA), isostearic acid, and 12-hydroxystearic acid.

Examples of the fluorine-based oil include perfluoropolyether, perfluorodecalin, and perfluorooctane.

### [Cosmetic Material and Optional Components Thereof]

The present invention relates to a cosmetic material containing the copolymer described above. Herein, a preferred range of the blending amount when blending the copolymer in which the aforementioned specified unreacted monomer is reduced into a cosmetic material is as described above, and it is possible to design or provide a cosmetic material in which the amount of the aforementioned specified unreacted monomer is 100 ppm or less.

The cosmetic material of the present invention can include various types of cosmetic raw materials in addition to the copolymer described above. The cosmetic material includes both a case in which the copolymer composition is produced in the form of the third aspect of the present invention and then compounded into a cosmetic material, and a case in which the copolymer and other cosmetic raw materials are separately compounded into the cosmetic material. These optional components are not particularly limited, and preferred examples include cosmetic materials and at least one type is selected from a group consisting of (B) water, (C) alcohols, (D) oil agents, (E) powders or coloring agents, (F) surfactants, (G) oil-soluble gelling agents, (H) organically modified clay minerals, (I) silicone resins, (J) silicone gums, (K) silicone elastomers, (L) organo-modified silicones, (M) ultraviolet light blocking components, and (N) water-soluble polymers.

Water (B) used in the cosmetic material according to the present invention is a medium of the cosmetic material, does not contain a component that is harmful to the human body, and is preferably clean. Examples include tap water, purified water such as distilled water and ion-exchanged water, mineral water, deep sea water, and the like. Furthermore, the form may be a physiological saline solution, phosphate buffered aqueous solution, or the like, if necessary.

Examples of alcohols (C) include the same compounds as described above.

Examples of oil agents (D) include the same compounds as described above.

### (E) Powder or Colorant

A powder or colorant, and particularly any powder used in cosmetic products (including powders and pigments used as colorants) can be further compounded into a cosmetic material containing a copolymer or copolymer composition of the present invention. Any powder or colorant can be used as long as the powder or colorant is one that is used in ordinary cosmetic materials, regardless of the shape (such as spherical, a rod-shape, needle-shape, plate-shape, sheet-shape, indefinite shape, spindle-shape, bowl-shape, and raspberry shape), the particle size (such as in an aerosol form, microparticle form, and pigment class) and the particle structure (such as porous, non-porous, and secondary aggregation) of the powder or colorant, and when these powders and/or colorants are compounded as a pigment, it is preferable to compound one or more kinds selected from inorganic pigment powder, organic pigment powder, and resin powder having an average particle diameter in the range of from1 nm to 20 µm.

Specific examples of the powder or colorant include inorganic powders, organic powders, surfactant metal salt powders (metal soaps), colored pigments, pearl pigments, metal powder pigments, and silicone elastomer powders; and composites of these can also be used. These powders or colorants include those which function as ultraviolet light blocking components.

Specifically, inorganic powders include titanium oxide, zirconium oxide, zinc oxide, cerium oxide, magnesium oxide, barium sulfate, calcium sulfate, magnesium sulfate, calcium sulfate, magnesium carbonate, talc, mica, kaolin, sericite, muscovite, synthetic mica, phlogopite, lepidolite, biotite, lithia mica, silicic acid, silicic anhydride, aluminum silicate, sodium silicate, sodium magnesium silicate, magnesium silicate, magnesium aluminum silicate, calcium silicate, barium silicate, strontium silicate, metal tungstate, hydroxyapatite, vermiculite, hydrargilite, bentonite, montmorillonite, hectorite, zeolite, ceramic powder, dicalcium phosphate, alumina, aluminum hydroxide, boron nitride, boron nitride, and the like; organic powders include polyamide powder, polyester powder, polyethylene powder, and polypropylene powder, polystyrene powder, polyurethane powder, polystyrene powder, benzoganamine powder, polymethylbenzoganamine powder, polytetrafluoroethylene powder, polymethyl methacrylate powder, cellulose, silk powder, nylon powder, 12-nylon, 6-nylon, silicone powder, silicone rubber powder, silicone elastomer spherical powder coated with polymethyl silsesquioxane thereon, polymethylsilsesquioxane spherical powder, styrene/acrylic acid copolymer, divinylbenzene/styrene copolymer, vinyl resin, urea resins, phenol resin, fluororesin, silicon resin, acrylic resin, melamine resin, epoxy resin, polycarbonate resin, microcrystalline fiber powder, starch powder, lauroyl lysine, and the like; surfactant metal salt powders include zinc stearate, aluminum stearate, calcium stearate, magnesium stearate, zinc myristate, magnesium myristate, zinc palmitate, zinc laurate, zinc cetyl phosphate, calcium cetyl phosphate, sodium zinc cetyl phosphate, and the like; colored pigments include inorganic red pigments such as red oxide, iron oxide, iron hydroxide, iron titanate, inorganic brown pigments such as γ-iron oxide, inorganic yellow pigments such as yellow iron oxide, black iron oxide, inorganic black pigments such as carbon black, inorganic purple pigments such as manganese violet, cobalt violet, and the like, inorganic green pigments such as chromium hydroxide, chromium oxide, cobalt oxide, cobalt titanate, and the like, inorganic blue pigments such as prussian blue, ultramarine blue, and the like; those obtained by laking tar dyes such as Red No. 3, Red No. 104, Red No. 106, Red No. 201, Red No. 202, Red No. 204, Red No. 205, Red No. 220, Red No. 226, Red No. 227, Red No. 228, Red No. 230, Red No. 401, Red No. 505, Yellow No. 4, Yellow No. 5, Yellow No. 202, Yellow No. 203, Yellow No. 204, Yellow No. 401, Blue No. 1, Blue No. 2, Blue No. 201, Blue No. 404, Green No. 3, Green No. 201, Green No. 204, Green No. 205, Orange No. 201, Orange No. 203, Orange No. 204, Orange No. 206, Orange No. 207, and the like, or those obtained by laking natural dyes such as carmine acid, lacquemic acid, carthamine, braziline, chrosine and the like; pearl pigments such as titanium oxide-coated mica, titanated mica, iron oxide-treated titanated mica, titanium oxide-coated mica, bismuth oxychloride, titanium oxide-coated bismuth oxychloride, titanium oxide-coated talc, fish scale foil, titanium oxide-coated colored mica and the like; oxidized titanium oxide coated mica, oxychlorinated bismuth, titanium oxide coated bismuth oxychloride, titanium oxide coated tantalum foil, fish scaly foil, titanium oxide coated colored mica, and the like; metal powder pigments such as aluminum, gold, silver, copper, platinum, stainless steel, and the like.

The silicone elastomer powder is, of the silicone elastomers (K) described below, a component in a powder form. These silicone elastomers are crosslinked products of a linear diorganopolysiloxane mainly composed of a diorganosiloxy unit (D unit), and can be suitably obtained by subjecting an organohydrogen polysiloxane having a silicon-bonded hydrogen atom in a side chain or at an end and a diorganopolysiloxane having an unsaturated hydrocarbon group such as an alkenyl group in a side chain or at an end to a crosslinking reaction in the presence of a hydrosilylation reaction catalyst. Since the silicone elastomer powder is soft, elastic, and excellent in oil absorption compared to the silicone resin powder composed of a T unit and a Q unit, the silicone elastomer powder can absorb oil and fat on the skin and prevent cosmetic collapse.

The silicone elastomer powder may have various shapes, such as spherical, flat, or indefinite. The silicone elastomer powder may be in the form of an oil dispersion. The cosmetic composition of the present invention is a silicone elastomer powder having a particle shape, a primary particle diameter thereof determined by observation using an electron microscope and/or an average primary particle diameter measured by laser diffraction/scattering method falls within a range of 0.1 to 50 µm, and a silicone elastomer powder having a spherical primary particle shape can be suitably compounded. The silicone elastomer constituting the silicone elastomer powder is preferably a silicone elastomer having a hardness of 80 or less, more preferably 65 or less according to JIS K 6253 "Hardness testing method for rubber, vulcanized or thermoplastic" as measured by type-A durometer.

Note that the silicone elastomer powder can also be used in the cosmetic material of the present invention in a form of an aqueous dispersion liquid. Examples of commercially available products of such an aqueous dispersion liquid include BY 29-129 and PF-2001 PIF Emulsion available from Dow Corning Toray Co., Ltd.

The silicone elastomer powder may optionally be subjected to a surface treatment with a silicone resin, silica, or the like. Examples of the surface treatment include those described in JP 2-243612 A, JP 8-12545 A, JP 8-12546 A, JP 8-12524 A, JP 9-241511 A, JP 10-36219 A, JP 11-193331 A, and JP 2000-281523 A. The silicone elastomer powder corresponds to the crosslinked silicone powder listed in the "Japanese Cosmetic Ingredients Codex". Commercial products of silicone elastomeric powders include, for example, Trefil E-506S, Trefil E-508, 9701 Cosmetic Powder, and 9702 Powder, available from Dow Corning Toray Co., Ltd.

Further, it is particularly preferable that a part or all of these powders or colorants are subjected to a water-repellency treatment. Such a treatment allows for stable compounding into the oil phase. Further, these powders or colorants may be combined with each other, and powders and colorants that have been subjected to a surface treatment using a general oil agent, a silicone compound other than the organopolysiloxane copolymer according to the present invention, or a fluorine compound or surfactant, etc. can also be used.

Examples of other water-repellency treatments include those in which the powder or colorant is treated with various water-repellency surface treatments, such as a methyl hydrogen polysiloxane treatment, a silicone resin treatment, a silicone gum treatment, an acrylic silicone treatment, an organosiloxane treatment such as a fluorinated silicone treatment, a metal soap treatment such as a zinc stearate treatment, a silane coupling agent treatment, a silane treatment such as an alkylsilane treatment, a fluorine compound treatment such as a perfluoroalkyl phosphate ester salt, a perfluoroether treatment, an amino acid treatment such as an N-lauroyl-L-lysine treatment, and an acrylic acid treatment such as a squalane treatment, and the like, and one or more of these treatments can be used in combination.

It is preferable that the above-mentioned powder or colorant is treated using another powder dispersant or surface treatment agent, in particular, the powder or colorant may be dispersed or surface treated by the novel powder treating agent and treatment method proposed by the present inventors in WO 2009/022621, JP 2011-148784 A, JP 2011-149017 A, JP 2011-246704 A, JP 2011-246705 A, JP 2011-246706 A, WO 2009/022621, WO 2011/049246, WO 2011/049248, JP 2011-286973, and the like, or the powder or colorant may be treated with the new powder treatment agent and the oiling agent to form a slurry. These novel treatment agents excel even further in performance areas such as dispersion stability and the effect of improving the inherent tactile sensation, and therefore, when these novel treatment agents are used in combination with the novel cosmetic raw materials of the present invention, an effect of further improving aspects such as the function, tactile sensation, and storage stability of the cosmetic material is anticipated.

Furthermore, some or all of these powders or colorants can be subjected to a hydrophilizing treatment. Through such treatment, the powder or colorant can be compounded in an aqueous phase.

Further, some or all of these powders or colorants can be subjected to a hydrophobizing treatment and a hydrophilizing treatment. Through such treatments, emulsification characteristics can be imparted to the powder itself. Examples of commercially available products include MZY-500SHE available from Tayca Corporation.

One or more types of the powder or colorant (E) in the copolymer composition or cosmetic material of the present invention can be used as necessary, and the blending amount thereof is not particularly limited. Furthermore, the powder or colorant (E) can be compounded within a range of 0.1 to 99.5 mass%, and preferably 1 to 99 wt.%, of the overall composition or cosmetic material. In particular, in the case of a powdery solid cosmetic material, the compounded amount is preferably in the range of from 80 to 99 mass% of the entire cosmetic material.

The cosmetic material containing the copolymer composition or copolymer of the present invention can contain a surfactant (F) as an optional component. As the surfactant (F), one or more types of surfactants can be used in combination according to the purpose, the types thereof selected from the group consisting of (F1) silicone-based surfactants, (F2) anionic surfactants, (F3) cationic surfactants, (F4) nonionic surfactants, (F5) amphoteric surfactants, and (F6) semipolar surfactants.

Examples of (F1) silicone-based surfactants include polyglyceryl-modified silicones, diglyceryl-modified silicones, glyceryl-modified silicones, sugar-modified silicones, fluoropolyether-modified silicones, polyether-modified silicones, carboxylic acid-modified silicones, linear silicone polyether block copolymers (such as polysilicone-13), long-chain alkyl-polyether comodified silicones, polyglyceryl-modified silicone elastomers, diglyceryl-modified elastomers, glyceryl-modified elastomers, and polyether modified elastomers. Furthermore, a silicone-based surfactant having an alkyl branch, linear silicone branch, or siloxane dendrimer branch, etc. applied as necessary at the same time as the hydrophilic group can be suitably used in the silicones or elastomers as described above. Commercially available products include SH 3771 M, SH 3772 M, SH 3773 M, SH 3775 M, BY 22-008M, BY 11-030, ES - 5373 FORMULATION AID, ES - 5612 FORMULATION AID, ES - 5300 FORMULATION AID, ES - 5600 SILICONE GLYCEROL EMULSIFIER (all are available from Dow Corning Toray Co., Ltd.).

Examples of the (F2) anionic surfactants include saturated or unsaturated fatty acid salts (such as sodium laurate, sodium stearate, sodium oleate, and sodium linolenate), alkyl sulfates, alkyl benzene sulfonates (such as hexyl benzenesulfonate, octyl benzenesulfonate, and dodecyl benzenesulfonate) and salts thereof, polyoxyalkylene alkyl ether sulfates, polyoxyalkylene alkenyl ether sulfates, polyoxyethylene alkyl sulfates, alkyl sulfosuccinates, alkyl polyoxyalkylene sulfosuccinates, polyoxyalkylene alkyl phenyl ether sulfates, alkane sulfonates, octyltrimethylammonium hydroxide, dodecyltrimethylammonium hydroxide, alkyl sulfonates, polyoxyethylene alkylphenyl ether sulfates, polyoxyalkylene alkyl ether acetates, alkyl phosphates, polyoxyalkylene alkyl ether phosphates, acyl glutamate, α-acyl sulfonate, alkyl sulfonates, alkyl allyl sulfonates, α-olefin sulfonate, alkylnaphthalene sulfonates, alkane sulfonates, alkyl or alkenyl sulfonates, alkyl or alkenyl sulfates, alkyl amide sulfates, alkyl or alkenyl phosphates, alkyl amide phosphates, alkyloyl alkyl taurine salt, N-acyl amino acid salt, sulfosuccinate, alkyl ether carboxylates, amide ether carboxylate, α-sulfo fatty acid ester salt, alanine derivatives, glycine derivatives, and arginine derivatives. Examples of the salts include an alkali metal salt such as a sodium salt, an alkaline earth metal salt such as a magnesium salt, an alkanolamine salt such as a triethanolamine salt, and an ammonium salt.

Examples of the (F3) cationic surfactants include alkyltrimethylammonium chloride, stearyltrimethylammonium chloride, lauryltrimethylammonium chloride, cetyltrimethylammonium chloride, beef tallow alkyltrimethylammonium chloride, behenyltrimethylammonium chloride, stearyltrimethylammonium bromide, behenyltrimethylammonium bromide, distearyldimethylammonium chloride, dichocoyl dimethylammonium chloride, dioctyldimethylammonium chloride, di (POE) oleylmethylammonium chloride (2EO), benzalkonium chloride, alkyl benzalkonium chloride, alkyl dimethyl benzalkonium chloride, benzethonium chloride, stearyl dimethyl benzyl ammonium chloride, lanolin-derived quaternary ammonium salt, diethylaminoethyl stearamide, diethylaminopropyl stearamide, behenic acid amidopropyldimethylhydroxypropylammonium chloride, stearoyl collamino formylmethyl pyridinium chloride, cetylpyridinium chloride, tall oil alkylbenzyl hydroxyethyl imidazolinium chloride, and benzylammonium salts.

Examples of the (F4) nonionic surfactants include polyglyceryl diisostearate, diglyceryl polyhydroxy stearate, isostearyl glyceryl ether, polyoxyalkylene ethers, polyoxyalkylene alkyl ethers, polyoxyalkylene fatty acid esters, polyoxyalkylene fatty acid diesters, polyoxyalkylene resin acid esters, polyoxyalkylene (hydrogenated) castor oils, polyoxyalkylene alkyl phenols, polyoxyalkylene alkyl phenyl ethers, polyoxyalkylene phenyl phenyl ethers, polyoxyalkylene alkyl esters, polyoxyalkylene alkyl esters, sorbitan fatty acid esters, polyoxyalkylene sorbitan alkyl esters, polyoxyalkylene sorbitan fatty acid esters, polyoxyalkylene sorbitol fatty acid esters, polyoxyalkylene glycerin fatty acid esters, polyglycerin alkyl ethers, polyglycerin fatty acid esters, sucrose fatty acid esters, fatty acid alkanolamides, alkyl glucosides, polyoxyalkylene fatty acid bisphenyl ethers, polypropylene glycols, diethylene glycols, polyoxyethylene/polyoxypropylene block polymers, alkyl polyoxyethylene/polyoxypropylene block polymer ethers, polyoxyethylene/polyoxypropylene block polymers, alkyl polyoxyethylene/polyoxypropylene block polymer ethers, and fluorine-based surfactants.

Examples of the (F5) amphoteric surfactants include imidazoline type, amidobetaine type, alkylbetaine type, alkylamidobetaine type, alkylsulfobetaine type, amidosulfobetaine type, hydroxysulfobetaine type, carbobetaine type, phosphobetaine type, aminocarboxylic acid type, and amido amino acid type amphoteric surfactants. Specific examples include imidazoline-type amphoteric surfactants such as sodium 2-undecyl-N,N-N-(hydroxyethyl carboxymethyl)-2-imidazoline, and 2-cocoyl-2-imidazolinium hydroxide-1-carboxyethyloxy disodium salt; alkyl betaine-type amphoteric surfactants such as lauryl dimethylaminoacetate betaine and myristyl betaine; amidobetaine amphoteric surfactants such as coconut oil fatty acid amide propyldimethylaminoacetic acid betaine, palm kernel oil fatty acid amido propyldimethylaminoacetic acid betaine, beef tallow fatty acid amido propyldimethylaminoacetic acid betaine, hydrogenated beef tallow fatty acid amido propyldimethylaminoacetic acid betaine, lauric acid amido propyldimethylaminoacetic acid betaine, myristic acid amido propyldimethylaminoacetic acid betaine, palmitic acid amido propyldimethylaminoacetic acid betaine, stearic acid amido propyldimethylaminoacetic acid betaine, and oleic acid amido propyldimethylaminoacetic acid betaine; alkyl sulfobetaine-type amphoteric surfactants such as coconut oil fatty acid dimethylsulfopropyl betaine; alkyl hydroxysulfobetaine-type amphoteric surfactants such as lauryl dimethyl amino hydroxysulfobetaine; phosphobetaine-type amphoteric surfactants such as lauryl hydroxyphosphobetaine; and amido amino acid-type amphoteric surfactants such as sodium N-lauroyl-N'-hydroxyethyl-N'-carboxymethylethylenediamine, sodium N-oleoyl-N'-hydroxyethyl-N'-carboxymethylethylenediamine, sodium N-cocoyl- N'-hydroxyethyl-N'-carboxymethylethylenediamine, potassium N-lauroyl-N'-hydroxyethyl-N'-carboxymethylethylenediamine, potassium N-oleoyl-N'-hydroxyethyl-N'-carboxymethylethylenediamine, sodium N-lauroyl-N-hydroxyethyl-N'-carboxymethylethylenediamine, sodium N-oleoyl-N-hydroxyethyl-N'-carboxymethylethylenediamine, sodium N-cocoyl-N-hydroxyethyl-N'-carboxymethylethylenediamine, monosodium N-lauroyl-N-hydroxyethyl-N',N'-dicarboxymethylethylenediamine, monosodium N-oleoyl-N- hydroxyethyl-N',N'-dicarboxymethylethylenediamine, monosodium N-cocoyl-N-hydroxyethyl-N',N'-dicarboxymethylethylenediamine, disodium N-lauroyl-N-hydroxyethyl-N',N'-dicarboxymethylethylenediamine, disodium N-oleoyl-N-hydroxyethyl-N',N'-dicarboxymethylethylenediamine, and disodium N-cocoyl-N-hydroxyethyl-N',N'-dicarboxymethylethylenediamine.

Examples of the (F6) semipolar surfactants include alkylamine oxide type surfactants, alkylamine oxides, alkylamidoamine oxides, and alkylhydroxyamine oxide, and alkyldimethylamine oxides having from 10 to 18 carbon atoms, alkoxyethyldihydroxyethylamine oxides having from 8 to 18 carbon atoms, and the like are preferably used. Specifically, dodecyl dimethylamine oxide, dimethyl octylamine oxide, diethyl decylamine oxide, bis-(2-hydroxyethyl) dodecylamine oxide, dipropyl tetradecylamine oxide, methyl ethyl hexadecylamine oxide, dodecyl amidopropyl dimethylamine oxide, cetyl dimethylamine oxide, stearyl dimethylamine oxide, tallow dimethylamine oxide, dimethyl-2-hydroxyoctadecylamine oxide, lauryl dimethylamine oxide, myristyl dimethylamine oxide, isostearyl dimethylamine oxide, coconut fatty acid alkyldimethylamine oxide, caprylic acid amidopropyldimethylamine oxide, capric acid amidopropyldimethylamine oxide, lauric acid amidopropyldimethylamine oxide, myristic acid amidopropyldimethylamine oxide, palmitic acid amidopropyldimethylamine oxide, stearic acid amidopropyldimethylamine oxide, isostearic acid amidopropyldimethylamine oxide, oleic acid amidopropyldimethylamine oxide, ricinoleic acid amidopropyldimethylamine oxide, 12-hydroxystearic acid amidopropyldimethylamine oxide, coconut fatty acid amidopropyldimethylamine oxide, palm kernel oil fatty acid amidopropyldimethylamine oxide, castor oil fatty acid amidopropyldimethylamine oxide, lauric acid amidoethyldimethylamine oxide, myristic acid amidoethyldimethylamine oxide, coconut fatty acid amidoethyldimethylamine oxide, lauric acid amidoethyldiethylamine oxide, myristic acid amidoethyldiethylamine oxide, coconut fatty acid amidoethyldiethylamine oxide, lauric acid amidoethyldihydroxyethylamine oxide, myristic acid amidoethyl dihydroxyethylamine oxide, and coconut fatty acid amide ethyl dihydroxyethylamine oxide are exemplified.

The amount of the surfactant (F) that is compounded in the emulsion composition of the present invention is not particularly limited. However, in order to stabilize the emulsion or dispersion, the surfactant (F) can be compounded in the emulsion composition or dispersion composition in a range of from 0.05 to 90 wt.%, preferably from 0.1 to 50 wt.%, and even more preferably from 0.5 to 25 wt.% per the weight of the composition.

### (G) Oil-Soluble Gelling Agent

Oil-soluble gelling agents are preferable and have a gelling or thickening effect. Specific examples include metal soaps such as aluminum stearate, magnesium stearate, and zinc myristate; amino acid derivatives such as N-lauroyl-L-glutamic acid, and α,γ-di-n-butylamine; dextrin fatty acid esters such as dextrin palmitate, dextrin stearate, dextrin 2-ethylhexanoic acid palmitate; sucrose fatty acid esters such as sucrose palmitate and sucrose stearate; benzylidene derivatives of sorbitol such as monobenzylidene sorbitol and dibenzylidene sorbitol; and the like. One or two or more of these can be used as necessary.

### (H) Organically Modified Clay Minerals

Examples of the organically modified clay mineral include dimethylbenzyldodecylammonium montmorillonite clay, dimethyldioctadecylammonium montmorinite clay, dimethylalkylammonium hectorite, benzyldimethyl stearylammonium hectorite, distearyldimethylammonium aluminum magnesium chloride-treated magnesium aluminum silicate, and the like. Commercially available products thereof include Benton 27 (hectorite treated with benzyl dimethyl stearyl ammonium chloride, available from National Lead Co.) and Benton 38 (hectorite treated with distearyl dimethyl ammonium chloride, available from National Lead Co.).

### (I) Silicone Resin

The silicone resin is an organopolysiloxane having a highly branched structure, a net-like structure, or a cage-like structure, and is in a liquid state or a solid state at room temperature, and may be any silicone resin commonly used in cosmetic materials as long as the silicone resin does not contradict the object of the present invention. Examples of solid silicone resins include MQ resin, MDQ resin, MTQ resin, MDTQ resin, TD resin, TQ resin, and TDQ resin made from optional combinations of triorganosiloxy units (M unit) (organo groups are methyl groups only, or are methyl groups and vinyl groups or phenyl groups), diorganosiloxy units (D unit) (organo groups are methyl groups only, or are methyl groups and vinyl groups or phenyl groups), monoorganosiloxy units (T unit) (organo groups are methyl groups, vinyl groups, or phenyl groups), and siloxy units (Q unit). Further, trimethyl siloxysilicate, polyalkyl siloxysilicate, trimethyl siloxysilicate containing dimethylsiloxy units, and alkyl (perfluoroalkyl) siloxysilicate are exemplified. It is particularly preferable that these silicone resins are oil-soluble and can be dissolved in D4 and D5.

The silicone resin forms a uniform film when applied to skin, hair, or the like, and provides a protective effect against drying and low temperature. Furthermore, the silicone resin having these branching units tightly adheres to the skin, hair, and the like, and can impart luster and a transparent feel to skin, hair, and the like.

### (J) Silicone Gum

In the present invention, ultra-high viscosity organopolysiloxanes, referred to as silicone gums, having a viscosity of greater than or equal to 1,000,000 mm²/s can also be used as silicone oils. Silicone gum is a linear diorganopolysiloxane with an ultra-high degree of polymerization, and is also referred to as a raw silicone gum or an organopolysiloxane gum. Silicone gums have a measurable degree of plasticity due to their high degree of polymerization, and are thereby distinguished from the oily silicones described above. Examples of such raw silicone rubbers include substituted or unsubstituted organopolysiloxanes having dialkylsiloxy units (D unit) such as dimethylpolysiloxane, methylphenylpolysiloxane, aminopolysiloxane, methylfluoroalkylpolysiloxane, and those having microcrosslinking structures thereof, and a representative example thereof is represented by the general formula: R¹⁰(CH₃)₂SiO{(CH₃)₂SiO)ₛ{(CH₃)R¹¹SiO}ₜSi(CH₃)₂R¹⁰ (wherein R¹¹ represents a vinyl group, a phenyl group, an alkyl having 6 to 20 carbon atoms, an aminoalkyl group having 3 to 15 carbon atoms, a perfluoroalkyl group having 3 to 15 carbon atoms, a quaternary ammonium salt group-containing alkyl group having 3 to 15 carbon atoms, and the end group R¹⁰ is a group selected from an alkyl group having 1 to 8 carbon atoms, a phenyl group, a vinyl group, an aminoalkyl group having 3 to 15 carbon atoms, a hydroxyl group and an alkoxy group having 1 to 8 carbon atoms, and s = 2,000 to 6,000, t = 0 to 1,000, s + t = 2,000 to 6,000). Among these, dimethylpolysiloxane raw rubbers having a degree of polymerization of 3000 to 20,000 are preferable. These silicone gums can be compounded as is, or as a liquid gum dispersion (oil dispersion of silicone gum) with the silicone gum dispersed in an oily silicone, into the cosmetic material according to the present invention.

Examples of such raw silicone rubbers include substituted or unsubstituted organopolysiloxanes having dialkylsiloxy units (D unit) such as dimethylpolysiloxane, methylphenylpolysiloxane, aminopolysiloxane, methylfluoroalkylpolysiloxane, and those having microcrosslinking structures thereof, and a representative example thereof is represented by the general formula: R¹⁰(CH₃)₂SiO{(CH₃)₂SiO)ₛ{(CH₃)R¹²SiO}ₜSi(CH₃)₂R¹⁰ (wherein R¹² represents a vinyl group, a phenyl group, an alkyl having 6 to 20 carbon atoms, an aminoalkyl group having 3 to 15 carbon atoms, a perfluoroalkyl group having 3 to 15 carbon atoms, a quaternary ammonium salt group-containing alkyl group having 3 to 15 carbon atoms, and the end group R¹⁰ is a group selected from an alkyl group having 1 to 8 carbon atoms, a phenyl group, a vinyl group, an aminoalkyl group having 3 to 15 carbon atoms, a hydroxyl group and an alkoxy group having 1 to 8 carbon atoms, and s = 2,000 to 6,000, t = 0 to 1,000, s + t = 2,000 to 6,000). Among these, dimethylpolysiloxane raw rubbers having a degree of polymerization of 3000 to 20,000 are preferable. Furthermore, an amino-modified methylpolysiloxane raw rubber having a 3-aminopropyl group, an N-(2-aminoethyl) 3-aminopropyl group, or the like in a side chain or at an end of the molecule is preferable. Additionally, in the present invention, one type or a combination of two or more types of silicone gums can be used as necessary.

Silicone gums have an ultra-high degree of polymerization, and therefore form a protective film having excellent residual properties on skin and hair and excellent air permeability. Thus, silicone gum is a component that can impart luster and gloss to particularly skin and hair, and can impart tension and a resilient texture to the entire skin and hair both during and after use.

The compounded amount of the silicone gum is, for example, in a range of from 0.05 to 30 wt.% (mass%), and preferably from 1 to 15 wt.% (mass%), of the total cosmetic material. Note that the silicone gum is easy to compound if used as an emulsion composition prepared through a pre-emulsification step (also including emulsion polymerization), and can be stably compounded in the cosmetic material of the present invention. If the compounded amount of the silicone gum is less than the abovementioned lower limit, the effect of imparting gloss to the skin and hair may be insufficient.

### (K) Silicone Elastomer

The silicone elastomer can be compounded into the cosmetic material in any form in accordance with the purpose thereof, but in particular, in addition to the silicone elastomer powder described in the aforementioned "(E) Powder", compounding the silicone elastomer as a crosslinkable organopolysiloxane is preferable. The silicone elastomer powder can be used in the cosmetic material of the present invention even in the form of an aqueous dispersion liquid. Examples of commercially available products of such an aqueous dispersion liquid include BY 29-129 and PF-2001 PIF Emulsion, available from Dow Corning Toray Co., Ltd. By compounding these silicone elastomer powders in the form of a water-based dispersion (= suspension), the usage feel of the cosmetic material of the present invention can be further improved, and thus such silicone elastomer powders are extremely useful.

As the crosslinkable organopolysiloxane, a non-emulsifying organopolysiloxane having a structure in which an organopolysiloxane chain is three-dimensionally crosslinked by reaction with a crosslinkable component or the like, and not having a hydrophilic part such as a polyoxyalkylene unit is preferable. Such crosslinkable organopolysiloxanes can be used without limitation irrespective of physical morphologies such as dilutions and properties, and of the preparation method, etc., and α,ω-diene crosslinked silicone elastomers described in US 5,654,362 (commercially available products include DC 9040 Silicone Elastomer Blend, DC 9041 Silicone Elastomer Blend, DC 9045 Silicone Elastomer Blend, and DC 9046 Silicone Elastomer Blend, available from Dow Corning Corporation, USA) are particularly preferred. Furthermore, a crosslinkable organopolysiloxane having fluidity at room temperature can also be suitably used, and an example thereof includes 3901 LIQUID SATIN BLEND (available from Dow Corning Corporation, USA).

### (L) Organo-Modified Silicone

The organo-modified silicone is preferably lipophilic. Specific examples include, in addition to the above, amino modified silicones, amino polyether modified silicones, epoxy modified silicones, carboxyl modified silicones, amino acid modified silicones, carbinol modified silicones, acrylic modified silicones, phenol modified silicones, amidoalkyl modified silicones, amino glycol modified silicones, and alkoxy modified silicones. The organo-modified silicone may have, in addition to the polysiloxane bond as the main chain, an alkylene chain, an aminoalkylene chain or a polyether chain of an extent such that the compound is not hydrophilic, and the organo-modified group may be present in one or both of a side chain and an end of the polysiloxane chain. When the cosmetic material of the present invention is used as a hair cosmetic material, amino modified silicones, carbinol modified silicones, aminopolyether modified silicones or aminoglycol modified silicones can be suitably used, and general examples include amino modified silicones having a 3-aminopropyl group, an N-(2-aminoethyl)3-aminopropyl group or the like.

Hereinafter, higher alkyl-modified silicones, alkyl-modified silicone resins, and polyamide-modified silicone resins that are particularly preferable as organo-modified silicones are described. The higher alkyl modified silicone is a component that is waxy at room temperature and is useful as a cosmetic raw material. Therefore, a higher alkyl modified silicone can be suitably used in the cosmetic material of the present invention. Examples of such higher alkyl-modified silicone waxes include methyl long-chain alkyl polysiloxanes capped at both ends of the molecular chain with trimethylsiloxy groups, dimethyl polysiloxane/methyl long-chain alkyl siloxane copolymers capped at both ends of the molecular chain with trimethylsiloxy groups, and long-chain alkyl-modified dimethyl polysiloxane at both ends of the molecular chain. Commercially available products of these include AMS-C30 Cosmetic Wax and 2503 Cosmetic Wax (available from Dow Corning Corporation, USA).

In the cosmetic material of the present invention, the higher alkyl modified silicone wax preferably has a melting point of 60°C or higher from the perspective of cosmetic retainability and high temperature stability.

The alkyl-modified silicone resin is a component that imparts sebum durability, moisture retention, and a smooth tactile sensation to a cosmetic material, and can be suitably used in a waxy form at room temperature. As the alkyl-modified silicone resin, for example, a silsesquioxane resin wax described in JP 2007-532754 T is preferable. Examples of commercially available products of alkyl-modified silicone resins include SW-8005 C30 RESIN WAX (available from Dow Corning Corporation, USA).

Examples of polyamide-modified silicones include the siloxane-based polyamide compounds described in US 5,981,680 (JP 2000-038450 A) and JP 2001-512164 T, and commercially available products thereof include 2-8178 Gellant and 2-8179 Gellant (available from Dow Corning Corporation, USA). Such polyamide-modified silicones also function as thickeners/gelling agents for oily raw materials, especially silicone oils.

### (M) Ultraviolet Light Blocking Component

The ultraviolet light blocking component includes inorganic ultraviolet light blocking components and organic ultraviolet light blocking components. If the cosmetic material of the present invention is a sunscreen cosmetic material, the cosmetic material preferably contains at least one inorganic or organic ultraviolet light blocking component, and especially an organic ultraviolet light blocking component. The copolymer of the present invention excels in miscibility with hexyl diethylaminohydroxybenzoyl benzoate known as "Uvinul A", bis-ethylhexyloxyphenol methoxyphenyl triazine known as "Tinosorb S", 2-ethylhexyl 2-cyano-3,3-diphenylprop-2-enoate known as "Octocrylene", and other cinnamic acid-based ultraviolet absorbing agents, and can improve the compounding stability with the copolymer of the present invention.

The inorganic ultraviolet light blocking component may be a component obtained by compounding the abovementioned inorganic pigment powder or metal powder pigment, etc. as an ultraviolet light dispersant, and examples of inorganic ultraviolet light blocking components include metal oxides such as titanium oxide, zinc oxide, cerium oxide, low-order titanium oxide, and iron-doped titanium oxide; metal hydroxides such as iron hydroxide; metal flakes such as plate-shaped iron oxide and aluminum flakes; and ceramics such as silicon carbide. Of these, the inorganic ultraviolet light blocking component is particularly preferably at least one selected from particulate, plate-shaped, needle-shaped or fibrous microparticle metal oxides and microparticle metal hydroxides, having an average particle diameter in the range of from 1 to 100 nm. It is preferable that these powders are conventionally subjected to known surface treatments, for example, a fluorine compound treatment (preferably a perfluoroalkyl phosphate treatment, perfluoroalkyl silane treatment, perfluoropolyether treatment, fluorosilicone treatment, or fluorinated silicone resin treatment), a silicone treatment (preferably a methyl hydrogen polysiloxane treatment, dimethyl polysiloxane treatment, or gas phase tetramethyl tetrahydrogen cyclotetrasiloxane treatment), a silicone resin treatment (preferably a trimethyl siloxysilicate treatment), a pendant treatment (adding an alkyl chain or such after a vapor phase process silicone treatment), a silane coupling agent treatment, a titanium coupling agent treatment, a silane treatment (preferably an alkylsilane or alkylsilazane treatment), an oil agent treatment, an N-acylated lysine treatment, a polyacrylic acid treatment, a metal soap treatment (preferably stearic acid or myristic acid salt), an acrylic resin treatment, or a metal oxide treatment, and these powders are preferably subjected to treatments with a plurality of these treatments. For example, the surface of the microparticulate titanium oxide may be coated with a metal oxide such as silicon oxide or alumina, followed by surface treatment with alkylsilane, or the like. The surface treatment amount is preferably in the range of 0.1 to 50 mass% in total with respect to the powder.

The organic ultraviolet light blocking component is a lipophilic ultraviolet light blocking component, for example, a benzoic acid-based ultraviolet absorber such as para-aminobenzoic acid (hereinafter abbreviated as PABA), PABA monoglycerin ester, N,N-dipropoxy PABA ethyl ester, N,N-diethoxy PABA ethyl ester, N,N-dimethyl PABA ethyl ester, N,N-dimethyl PABA butyl ester, and hexyl diethylaminohydroxybenzoyl benzoate; an anthranilic acid-based ultraviolet absorber such as homomenthyl-N-acetylanthranilate; a salicylic acid-based ultraviolet absorber such as amyl salicylate, menthyl salicylate, homomenthyl salicylate, octyl salicylate, phenyl salicylate, benzyl salicylate, and p-isopropanol phenyl salicylate; a cinnamic acid-based ultraviolet absorber such as octyl cinnamate, ethyl-4-isopropyl cinnamate, methyl-2,5-diisopropyl cinnamate, ethyl-2,4-diisopropyl cinnamate, methyl-2,4-diisopropyl cinnamate, propyl-p-methoxy cinnamate, isopropyl-p-methoxy cinnamate, isoamyl-p-methoxy cinnamate, octyl-p-methoxy cinnamate (2-ethylhexyl-p-methoxy cinnamate), 2-ethoxyethyl-p-methoxy cinnamate, cyclohexyl-p-methoxy cinnamate, ethyl-α-cyano-β-phenyl cinnamate, 2-ethylhexyl-α-cyano-β-phenyl cinnamate, glyceryl mono-2-ethylhexanoyl-diparamethoxy cinnamate, and 3-methyl-4-[methylbis (trimethylsiloxy) silyl] butyl 3,4,5-trimethoxycinnamate; a benzophenone-based ultraviolet absorber such as 2,4-dihydroxybenzophenone, 2,2'-dihydroxy-4-methoxybenzophenone, 2,2'-dihydroxy-4,4'-dimethoxybenzophenone, 2,2',4,4'-tetrahydroxybenzophenone, 2-hydroxy-4-methoxybenzophenone, 2-hydroxy-4-methoxy-4'-methylbenzophenone, 2-hydroxy-4-methoxybenzophenone-5-sulfonate, 4-phenylbenzophenone, 2-ethylhexyl-4'-phenyl-benzophenone-2-carboxylate, hydroxy-4-n-octoxybenzophenone, and 4-hydroxy-3-carboxybenzophenone; 3-(4'-methylbenzylidene)-d,l-camphor, 3-benzylidene-d,l-camphor, urocanic acid, ethyl urocanate, 2-phenyl-5-methylbenzoxazole, 2,2'-hydroxy-5-methylphenylbenzotriazole, 2-(2'-hydroxy-5'-tert-octylphenyl) benzotriazole, 2-(2'-hydroxy-5'-methylphenylbenzotriazole, dibenzalazine, dianisoylmethane, 4-methoxy-4'-tert-butyldibenzoylmethane, and 5- (3,3-dimethyl-2-norbornylidene)-3-pentan-2-one.

It is also possible to use a polymer powder containing the organic ultraviolet light blocking component in a hydrophobic polymer powder. The polymer powder may or may not be hollow, the average primary particle size thereof may be in the range of 0.1 to 50 µm, and the particle size distribution may be broad or sharp. Examples of the polymer include acrylic resin, methacrylic resin, styrene resin, polyurethane resin, polyethylene, polypropylene, polyethylene terephthalate, silicone resin, nylon, acrylamide resin, and silylated polypeptide resin. A polymer powder containing an organic ultraviolet light blocking component in a range of from 0.1 to 30 mass% is preferable, and a polymer powder containing 4-tert-butyl-4'-methoxydibenzoylmethane, which is a UV-A absorber, is particularly preferable.

A polymer powder having the organic ultraviolet light blocking component dispersed in water can be used. Commercially available products include Tinosorb A2B (available from BASF).

In the cosmetic material of the present invention, the ultraviolet light blocking component which can be suitably used is at least one selected from the group consisting of microparticulate titanium oxide, microparticulate zinc oxide, 2-ethylhexyl paramethoxycinnamate, 4-tert-butyl-4'-methoxydibenzoylmethane, hexyl diethylamino hydroxybenzoylbenzoate, bis-ethylhexyloxyphenol methoxyphenyl triazine, 2-ethylhexyl 2-cyano-3,3-diphenylpropa-2-enoate, and other benzophenone-based ultraviolet absorbers. These ultraviolet light blocking components are generally used, are easily available, and have a high ultraviolet light blocking effect, and therefore can be suitably used. In particular, it is preferable to use a combination of an inorganic-based and an organic-based ultraviolet light blocking component, and it is more preferable to use a combination of an ultraviolet light blocking component corresponding to UV-A and an ultraviolet light blocking component corresponding to UV-B.

### (N) Water-Soluble Polymer

Meanwhile, the cosmetic material of the present invention may be an aqueous or emulsion type cosmetic material containing a large amount of water-soluble components, and a water-soluble polymer (N) may be compounded in accordance with the dosage form thereof, and is preferable. As the water-soluble polymer, one or more water-soluble polymers can be used. Examples of natural water-soluble polymers include gum arabic, tragacanth gum, galactan, guar gum, carob gum, karaya gum, carrageenan, pectin, cantene, quince seed (quince), algecolloid (brown algae extract), starch (rice, corn, potato, and wheat), glycyrrhetinic acid, and other plant-based polymers; microbial polymers such as xanthan gum, dextran, succinoglucan, and pullulan; and animal-based polymers such as collagen, casein, albumin, and gelatin. Examples of semi-synthetic water-soluble polymers include starch-based polymers such as carboxymethyl starch and methylhydroxypropyl starch; methyl cellulose, nitrocellulose, ethyl cellulose, methylhydroxypropyl cellulose, hydroxyethyl cellulose, sodium cellulose sulfate, hydroxypropyl cellulose, sodium carboxymethyl cellulose (CMC), crystalline cellulose, cellulose powder, and other such cellulose-based polymers; and alginate-based polymers such as sodium alginate and propylene glycol alginate. Examples of synthetic water-soluble polymers include polyvinyl alcohol, polyvinyl methyl ether-based polymers, polyvinyl pyrrolidone, carboxyvinyl polymers (CARBOPOL 940, 941; BF Goodrich), and other vinyl-based polymers; polyethylene glycol 20,000, polyethylene glycol 6000, polyethylene glycol 4000, and other such polyoxyethylene-based polymers; polyoxyethylene-polyoxypropylene copolymers, PEG/PPG methyl ethers, and other copolymer-based polymers; acrylic polymers such as sodium polyacrylate, polyethyl acrylate, and polyacrylamide; and polyethyleneimine, and cationic polymers. Examples of other cationic water-soluble polymers include, particularly as components that can be favorably compounded in hair cosmetic materials, quaternary nitrogen-modified polysaccharides (e.g., cationically modified cellulose, cationically modified hydroxyethyl cellulose, cationically modified guar gum, cationically modified locust bean gum, and cationically modified starch, etc.), dimethyl diallyl ammonium chloride derivatives (e.g., dimethyl diallyl ammonium chloride-acrylamide copolymers, and polydimethyl methylene piperidinium chloride, etc.), and vinylpyrrolidone derivatives (e.g., a vinylpyrrolidone-dimethylamino ethylmethacrylate copolymer salt, a vinylpyrrolidone-methacrylamide propyltrimethyl ammonium chloride copolymer, and a vinylpyrrolidone-methylvinyl imidazolium chloride copolymer, etc.).

Other components ordinarily used in cosmetic materials can be added to the cosmetic material of the present invention within a range that does not hinder the effect of the present invention, and examples of other such components include: organic resins, moisturizing agents, antiseptic agents, antimicrobial agents, perfumes, salts, antioxidants, pH adjusting agents, chelating agents, refreshing agents, anti-inflammatory agents, skin beautifying components (skin lightening agents, cell activating agents, rough skin improving agents, circulation promoters, skin astringents, anti-seborrheic agents, etc.), vitamins, amino acids, nucleic acids, hormones, and inclusion compounds. These specific examples are common with, but not limited to, those examples specifically disclosed in paragraphs [0100] to [0113], etc. of JP 2011-149017 A.

The cosmetic material of the present invention can contain a natural plant extract component, a seaweed extract component, and an herbal medicine component in accordance with the purpose thereof. Two or more types of these components may be compounded. These specific examples are common with, but not limited to, those examples specifically disclosed in paragraph [0115], etc. of JP 2011-149017 A.

Depending on the purpose thereof, the cosmetic material of the present invention may contain, for example, a solvent such as light isoparaffin, ether, LPG, N-methylpyrrolidone, alternative chlorofluorocarbons, and the like.

Furthermore, in addition to the copolymer of the present invention, an alkyl-modified silicone resin wax can be used in the cosmetic material of the present invention. These are film-forming components similar to the copolymers of the present invention.

As the alkyl-modified silicone resin wax, for example, silsesquioxane resin wax described in JP 2007-532754 T is preferable.

### [Type and Formulation of Cosmetic Material]

Furthermore, the type of cosmetic material and a form of a formulation thereof are not particularly limited, and may be skin cosmetic materials such as skin care products, antiperspirant products, deodorant products, makeup products, ultraviolet light blocking product, and the like; eyelash cosmetic products, hair cleaning agent products, hair dressing products, hair coloring products, hair nourishing products, hair rinsing products, hair conditioner products, hair treatment products, and other head hair cosmetic products; and hair cosmetic products such as bath cosmetic products and the like. Furthermore, the form thereof is not particularly limited, but may be a solution, emulsion, cream, solid, semi-solid, paste, gel, powder, multilayer, mousse, water-in-oil or oil-in-water emulsified composition (emulsion composition).

Herein, topical agents are applied to the skin, nails, hair, and the like of the human body, and can be used to treat various diseases by blending a pharmaceutical active component, for example. The cosmetic material is also applied to the skin, nails, hair, and the like of the human body, but are used for cosmetic purposes. Even if the application is a "topical agent", use in reality is possible in the same dosage and administration as a cosmetic material. Therefore, in the cosmetic material in the present application, such topical agents are also described as being included as cosmetic materials. Examples thereof include antiperspirants, skin cleaning agents, skin topical agents, hair cleaning agents, hair topical agents, and the like. Examples of applications as a drug of the topical agent include, but are not limited to, hair growing agents, hair tonics, analgesics, disinfectants, anti-inflammatory agents, refreshing agents, and skin anti-aging agents.

### Film-Forming Properties, Effect on Feel on Hair

Since the cosmetic material according to the present invention contains the aforementioned copolymer, a film having excellent water repellency as well as excellent durability and sebum durability can be formed on skin or hair. In particular, the copolymer of the present invention has an extremely low amount of the monomer containing a carbosiloxane dendrimer structure, having properties which are of concern for irritation and which are difficult to separate or remove from the copolymer by ordinary distilling means, is capable of forming on the skin or hair a film having excellent water resistance and sebum resistance, and can design a cosmetic material that provides these functional films without irritating properties.

The skin cosmetic materials can be used on any part of the scalp, face (including lips, eyebrows, and cheeks), fingers, nails, and entire body. More specific examples include skin cleansing agent products such as cleansing gels, cleansing creams, cleansing foams, facial cleansing creams, eye makeup removers, facial cleansing foams, liquid soaps (body soaps), hand soaps, gelatinous soaps, shaving creams, removers, and anti-acne cosmetics; skincare products such as skin creams, scalp treatments, skin milks, milk lotions, emulsions, facial packs, body powder, essences, shaving lotions, and massage lotions; makeup products such as foundations, liquid foundations, oily foundations, makeup bases, white powders, face powders, blushes, lip creams, rouge, lip gloss, eye-creams, mascara, eyebrow pencils, and eyelash cosmetic products; antiperspirants such as deodorants; and UV light blocking products such as sunscreens and sunburn medicinal agents (suntanning agents).

Examples of the hair cosmetic products include hair detergents such as shampoos and rinse-in-shampoos; hair dressing products such as hair waxes, hair curl holding agents, setting agents, hair creams, hair sprays, and hair liquids; hair coloring products such as hair dyeing agents, hair color sprays, hair color rinses, and hair color sticks; hair tonic products such as hair tonics, hair treatment essences, and hair packs; and hair-rinse or hair conditioning products such as oil rinses, cream rinses, treatment rinses, hair conditioners, and hair treatments. The bath cosmetic products include a foam bath.

[Design and Guidelines for Cosmetic Formulations Containing the Copolymer of the Present Invention]

The copolymer according to the present invention has the same water repellency, washability, film-forming properties, and other properties as a known acrylic-silicone dendrimer copolymer, except that the amount of the monomer containing a carbosiloxane dendrimer structure, having properties which are of concern for irritation and which are difficult to separate or remove from the copolymer by ordinary distilling means is extremely small. For this reason, for example, some or all of the vinyl-based polymer having a carbosiloxane dendrimer structure in a side chain as described in Patent No. 4009382 (Japanese Unexamined Patent Application 2000-063225) and the like can be substituted in known formulations. In other words, with regard to a cosmetic formulation containing a vinyl-based polymer having a known carbosiloxane dendrimer structure in a side chain, the present applicant clearly instructs herein to design or manufacture the cosmetic formulation by substituting the corresponding vinyl-based polymer with the copolymer of the present invention.

Similarly, the copolymer of the present invention may be used by substituting some or all in known formulations proposed for commercial products of vinyl-based polymers having a carbosiloxane dendrimer structure in a side chain. Examples of commercial products include FA4001 CM Silicone Acrylate, FA4002 ID Silicone Acrylate and DOWSIL(TM) FA4003 DM Silicone Acrylate, and the like manufactured by Dow Toray Co., Ltd. Cosmetic formulations using these products are known in a number of products, patents, formulation samples provided by DOW SILICONE CORPORATION, public information at IP.com, and the like, and the copolymer according to the present invention may be used to replace some or all of the vinyl-based polymer having a carbosiloxane dendrimer structure in a side chain in the products without any restriction, which is preferred.

When the copolymer of the present invention replaces a vinyl-based polymer having a carbosiloxane dendrimer structure in a side chain in a known cosmetic formulation, there is a benefit where a corresponding material in the cosmetic formulation can be replaced by the copolymer, based on a normal compounding formulation of a person of ordinary skill in the art, and a benefit of being able to provide a cosmetic material in which the amount of the monomer containing a carbosiloxane dendrimer structure, having properties which are of concern for irritation and which are difficult to separate or remove from the copolymer by ordinary distilling means, is reduced or is essentially not included, using a known formulation and without impairing the performance and properties of the cosmetic material.

The copolymer according to the present invention can also be used in other applications and compounded into other products besides cosmetic materials, such as in various external preparations, paints, coating agents, antifoaming agents, and deodorants.

### <Film-Forming Agent>

The copolymer film-forming agent according to the present invention can be used in applications other than cosmetic materials. In other words, the film can be used in any field so long as the application requires a water repellency effect, oil repellency effect, integrity, strength and scratch resistance on a film. In particular, use is possible as an agent for imparting water repellent effects to paint compositions and fibers, and as an agent for treating inorganic and organic materials. Furthermore, the film-forming agent of the present invention can be, if necessary, used in an aqueous environment, and therefore is easy to handle.

Specifically, the film-forming agent of the present invention has excellent compounding stability and does not impair film-forming properties and usability, and is therefore preferable for use as a film-forming agent for industrial applications in textile treatment agents or fabric and textile treatment agents.

### [Method of Manufacturing the Copolymer According to the Present Invention]

As described above, the monomer containing a carbosiloxane dendrimer structure, which is a raw material for the copolymer according to the present invention, has properties where separation or removal is difficult from the copolymer by ordinary distillation means. However, with the method of manufacturing the copolymer according to the present invention, the monomer can be reduced to 2500 ppm or less for the copolymer. Furthermore, the manufacturing method having the following step (I) can reduce the reaction time required for the radical polymerization reaction and thus improve industrial production efficiency.

A method of manufacturing the copolymer according to the present invention, includes:
a step of adding a polymerization initiator to (a1) the unsaturated monomer having a carbosiloxane dendrimer structure having a radically polymerizable organic group, and
(a2) the unsaturated monomer having a radically polymerizable vinyl group, which is different from component (a1)
to perform a radical polymerization reaction; and
step (I): a step (Ia) of further adding the same or different polymerization initiator than the initially added polymerization initiator at least two hours after adding the initial polymerization initiator for the radical polymerization reaction. The manufacturing method may and preferably includes: a step (Ib) of adding a monomer (a2') having a radically polymerizable vinyl group with a boiling point less than 160°C at ambient pressure (1 atm) to advance the radical polymerization reaction of unreacted component (a1), optionally in step (I); and step (II): a step of distilling off unreacted unsaturated monomers or saturated monomers remaining in the system at ambient pressure or under reduced pressure after step (I).

Herein, component (a1), component (a2) and the polymerization initiator are as described above. Furthermore, a monomer (a2') having a radically polymerizable vinyl group with a boiling point less than 160°C at ambient pressure (1 atm) is not particularly limited so long as the properties are provided, and particularly preferred examples of component (a2) include methyl (meth)acrylate, ethyl (meth)acrylate, and the like, which have low boiling points and are easily distilled at reduced pressure. Furthermore, the radical polymerization reaction may be carried out in the reaction solvent described above, and the reaction solvent may and is preferably solventsubstituted by step (IV) described below.

Step (I) described above is a step required to improve the reaction efficiency of the unsaturated monomer having a carbosiloxane dendrimer structure having the radical polymerizable organic group (a1) to be reduced and to control the residual amount thereof, and is a step where, at least two hours after adding a first polymerization initiator in the radical polymerization reaction, a polymerization initiator that is the same as or different from the first added polymerization initiator is further added. By introducing an additional polymerization initiator into the reaction system at a time interval from initially adding the polymerization initiator, specifically at least two hours, and preferably two and a half hours or more later, the radical polymerization reaction of component (a1) which was not expended in the initial radical polymerization reaction proceeds, and the residual amount in the system is significantly reduced. Herein, the amount of the polymerization initiator to be further added is not particularly limited, but may be within a range of 0.5 to 5.0 equivalents, and particularly preferably within a range of 0.6 to 4.5 equivalents, relative to the initially added polymerization initiator. Furthermore, the reaction time required for the radical polymerization reaction can be reduced and the industrial production efficiency of the copolymer according to the present invention can be improved by adding an additional polymerization initiator at a time interval from initially adding the polymerization initiator.

Furthermore, the aforementioned step (I) may further optionally include a step (Ib) of adding a monomer (a2') having a radically polymerizable vinyl group with a boiling point less than 160°C at ambient pressure (1 atm) to advance the radical polymerization reaction of unreacted component (a1). By introducing an additional polymerization initiator and another reactive unsaturated monomer into the reaction system at a time interval from initially adding the polymerization initiator, the radical polymerization reaction of component (a1) which was not expended in the initial radical polymerization reaction proceeds along with the additional component (a2'), and the residual amount in the system is significantly reduced. Furthermore, component (a2') has a low boiling point, and therefore can be easily distilled off at reduced pressure after reacting component (a1).

By the aforementioned step (I), the saturated monomer, which is component (a1) or a hydrogenated product thereof having properties of being difficult to separate and remove from the copolymer by means such as reduced pressure distillation or the like, is efficiently reacted and expended in advance, such that the residual amount thereof in the system is significantly reduced.

The manufacturing method of the present invention may further have step (II): a step of distilling off unreacted unsaturated monomers or saturated monomers remaining in the system at ambient pressure or under reduced pressure after step (I) described above. Note that the purpose of this step is to purify the unreacted monomer by removing a component with a low boiling point, and the amount of component (a1) has already been reduced in step (I), which is a preliminary step thereof. Distillation of the unreacted unsaturated monomer or saturated monomer under ambient pressure or reduced pressure in step (II) can be carried out without particular limitation using a known method such as stripping or the like.

Note that refluxing, reprecipitation, filtration, or other step may be performed during, before, or after a subsequent step after step (I).

The manufacturing method of the present invention may include, after the aforementioned step (I), and before or simultaneously with the step (II), a step (III): a step of performing a hydrogenation reaction on the unreacted unsaturated monomer remaining in the system to convert the unsaturated monomer into a saturated monomer. The step proceeds by contacting the polymerization reaction product of step (I) with the hydrogenation reaction catalyst (nickel catalyst or palladium catalyst) to form a saturated monomer, which is a hydrogenation reaction product of the unreacted component (a1) or the like, and to reduce the odor of the copolymer. Note that step (III) is a step of converting the unreacted component (a1) into a saturated monomer, and therefore, the amount of monomers derived from the unreacted component (a1) is not reduced in the aforementioned step (III).

The manufacturing method of the present invention preferably includes, at an arbitrary timing, and preferably before or simultaneously with the aforementioned step (II), step (IV): a step of adding one or more types of (C) alcohols and/or (D) oil agents as dispersants to the system and removing the reaction solvent for the radical polymerization reaction. With this step, a copolymer composition containing alcohols (C) and/or oil agents (D) having excellent blending stability and handling workability for use in cosmetic materials and the like can be obtained. The reaction solvent for the radical polymerization reaction can be removed without particular limitation by using a known method such as stripping or the like under ambient pressure or reduced pressure, as in step (II) described above, and can be replaced with one or more of alcohols (C) and/or oil agents (D) described above. Note that when the reaction solvent for the radical polymerization reaction is an alcohol (C), the radical polymerization reaction solution itself forms a copolymer composition containing alcohols (C), and thus can be used as is as a cosmetic raw material or film-forming agent, if desired.

### Examples

Hereinafter, the present invention is described in greater detail through examples; but the present invention is not limited thereto.

Each characteristic in the examples and the like was measured by the following methods.

### [GPC Peak Top Molecular Weight]

GPC analysis was performed using toluene as an eluting solvent and TSKgel Multipore HXL-M on a column, and the molecular weight was determined using a calibration curve with standard polystyrene.

### [Quantitative Determination of Residual Carbosiloxane Dendrimer Monomer (A-1)]

Quantification was performed on high performance liquid chromatography (Shimadzu Corporation, Prominence-i LC-2030C3D, column: Atlantis T3-3 µm (3.0 x 100 mm), UV visible absorption detecting part, eluent solvent: water/methanol/THF gradient mode).

### [Dynamic Viscosity]

The viscosity of the compositions was measured at 25°C using a Uveraude viscosity tube.

### [Nonvolatile Content]

Determined from the amount of sample remaining after 1 g of the sample was weighed into an aluminum dish having a diameter of 6 cm, and then heated at 150°C for 1 hour.

### [Film Stickiness]

A film prepared on an aluminum dish in the same manner as described above was touched with a finger, and whether or not the aluminum dish integrated with the film could be lifted was determined.

### [Contact Angle (Water)]

An IPA solution of a vinyl-based copolymer was coated onto a glass plate, after which the solvent was removed by drying at room temperature to obtain a coating film of a vinyl-based polymer. A 5 µL water droplet was placed on the coating film surface, and the contact angle with the water was measured. A drop shape analysis system (KRUSS DSA10 Mk-2) was used as the measurement device, and an average value of n = 5 or greater was determined.

### [Contact Angle (Artificial Sebum)]

An IPA solution of a vinyl-based copolymer was coated onto a glass plate, after which the solvent was removed by drying at room temperature to obtain a coating film of a vinyl-based polymer. A 5 µL droplet of artificial sebum (triolein:oleic acid:squalane = 3:1:1 mixture) was placed on the coating film surface, and the contact angle with respect to the artificial sebum was measured. A drop shape analysis system (KRUSS DSA10 Mk-2) was used as the measurement device, and an average value of n = 5 or greater was determined.

### [Example 1]

### [Preparation of the Copolymer and a Liquid Composition Thereof]

163 g of isopropyl alcohol (IPA) was inserted into a 1 liter four-neck flask equipped with a stirring device, a thermometer, and a reflux tube. The mixture was bubbled with nitrogen gas, and then sufficiently degassed and heated to 70°C. 110 g (55% by weight) of methyl methacrylate, 10 g (5% by weight) of n-butyl acrylate, 80 g (40% by weight) of a carbosiloxane dendrimer monomer as expressed by the following formula (A-1):

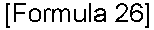

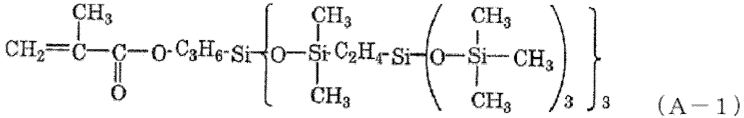

4.4 g (2.2% by weight) of methyl 2,2'-azobis-2-isobutyrate (V-601, manufactured by Wako Pure Chemical Industries, Ltd.), and 107 g of IPA were introduced and dissolved in a dripping funnel.

In a nitrogen atmosphere, the monomer mixture was dripped through a dripping funnel over 1 hour while being maintained at 70°C. After dripping was completed, heating and stirring were performed for 3 hours under a nitrogen atmosphere, and then a solution of 4.4 g (2.2% by weight) of V-601 dissolved in 30 g of IPA was added. After further stirring for 9 hours, a reaction product with a nonvolatile content of 40.8% was obtained. Analysis by GPC revealed that the polymer had a peak top molecular weight of 15,900. Furthermore, analysis by high-performance liquid chromatography revealed that 1,330 ppm of carbosiloxane dendrimer monomer (A-1) remained.

### [Reference Example 1]

163 g of IPA was inserted into a 1 liter four-neck flask equipped with a stirring device, a thermometer, and a reflux tube. The mixture was bubbled with nitrogen gas, and then sufficiently degassed and heated to 70°C. 110 g (55% by weight) of methyl methacrylate, 10 g (5% by weight) of n-butyl acrylate, 80 g (40% by weight) of the carbosiloxane dendrimer monomer (A-1), 2 g (1% by weight) of V-601, and 107 g of IPA were introduced and dissolved in a dripping funnel. In a nitrogen atmosphere, the monomer mixture was dripped through a dripping funnel over 1 hour while being maintained at 70°C. After dripping was completed, heating and stirring were performed for 8 hours under a nitrogen atmosphere to obtain a reaction product with a nonvolatile content of 40.6%. Analysis by GPC revealed that the polymer had a peak top molecular weight of 23.600. Furthermore, analysis by high-performance liquid chromatography revealed that 30,750 ppm of carbosiloxane dendrimer monomer (A-1) remained.

### [Reference Example 2]

A reaction product was obtained by a similar operation except that the amount of V-601 in Reference Example 1 was changed to 4.4 g (2.2% by weight). Analysis by GPC revealed that the polymer had a peak top molecular weight of 16,000. Furthermore, analysis by high-performance liquid chromatography revealed that 11,000 ppm of carbosiloxane dendrimer monomer (A-1) remained.

### [Reference Example 3]

A reaction product was obtained by a similar operation except that the amount of V-601 in Reference Example 1 was changed to 8.8 g (4.4% by weight). Analysis by GPC revealed that the polymer had a peak top molecular weight of 12,600. Furthermore, analysis by high-performance liquid chromatography revealed that 2,250 ppm of carbosiloxane dendrimer monomer (A-1) remained.

### [Summary 1]

The aforementioned results are summarized in Table 1. From the comparisons between Example 1 with Reference Example 2 and Reference Examples 1 to 3, it was found that the polymer molecular weight was determined based on the amount of the polymerization initiator V-601 added first. The residual amount of the carbosiloxane dendrimer monomer (A-1) with inferior reactivity could be significantly reduced by additionally adding the polymerization initiator V-601 with a time difference.

**[Table 1]**

| | | | Examples | Reference Examples | | |
|---|---|---|---|---|---|---|
| | | | 1 | 1 | 2 | 3 |
| Copolymer | Composition | Methyl methacrylate | 55 | 55 | 55 | 55 |
| | | N-butyl acrylate | 5 | 5 | 5 | 5 |
| | | A-1 | 40 | 40 | 40 | 40 |
| | | Polymerization initiator V-601 | 2.2 | 1 | 2.2 | 4.4 |
| | | Polymerization initiator V-601 (post addition) | 2.2 | - | - | - |
| | Reaction | Polymerization time after dripping V-601 | 3 | 8 | 8 | 8 |
| | | Polymerization time after adding V-601 | 9 | - | - | - |
| | Analysis | GPC peak top molecular weight | 15,900 | 23,600 | 16,000 | 12,600 |
| | | Residual A-1 (ppm) | 1,330 | 30,750 | 11,000 | 2,250 |

### [Example 2]

163 g of IPA was inserted into a 1 liter four-neck flask equipped with a stirring device, a thermometer, and a reflux tube. The mixture was bubbled with nitrogen gas, and then sufficiently degassed and heated to 70°C. 110 g (55% by weight) of methyl methacrylate, 10 g (5% by weight) of n-butyl acrylate, 80 g (40% by weight) of the carbosiloxane dendrimer monomer (A-1), 4.4 g (2.2% by weight) of V-601, and 107 g of IPA were introduced and dissolved in a dripping funnel. In a nitrogen atmosphere, the monomer mixture was dripped through a dripping funnel over 1 hour while being maintained at 70°C. After dripping was completed, heating and stirring were performed for 6 hours under a nitrogen atmosphere, and then a solution of 0.6 g (0.3% by weight) of V-601 dissolved in 30 g of IPA was added. After further stirring for 6 hours, a reaction product with a nonvolatile content of 40.0% was obtained. Analysis by GPC revealed that the polymer had a peak top molecular weight of 17,200. Furthermore, analysis by high-performance liquid chromatography revealed that 2,350 ppm of carbosiloxane dendrimer monomer (A-1) remained.

### [Comparative Example 1]

A reaction product was obtained by a similar operation except that the amount of V-601 in Reference Example 1 was 4.4 g (2.2% by weight) and the heating and stirring time was 12 hours. Analysis by GPC revealed that the polymer had a peak top molecular weight of 17,000. Furthermore, analysis by high-performance liquid chromatography revealed that 5,200 ppm of carbosiloxane dendrimer monomer (A-1) remained.

### [Comparative Example 2]

A reaction product was obtained by a similar operation except that the amount of V-601 in Reference Example 1 was 4.4 g (2.2% by weight) and the heating and stirring time was 24 hours. Analysis by GPC revealed that the polymer had a peak top molecular weight of 18,000. Furthermore, analysis by high-performance liquid chromatography revealed that 2,740 ppm of carbosiloxane dendrimer monomer (A-1) remained.

### [Summary 2]

The aforementioned results are summarized in Table 2. It was found that adding the initiator not only reduced the residual amount of the carbosiloxane dendrimer monomer (A-1) with inferior reactivity, but also significantly reduced the reaction time.

**[Table 2]**

| | | | Examples | | Comparative Examples | |
|---|---|---|---|---|---|---|
| | | | 1 | 2 | 1 | 2 |
| Copolymer | Composition | Methyl methacrylate | 55 | 55 | 55 | 55 |
| | | N-butyl acrylate | 5 | 5 | 5 | 5 |
| | | A-1 | 40 | 40 | 40 | 40 |
| | | Polymerization initiator V-601 | 2.2 | 2.2 | 2.2 | 2.2 |
| Analysis | | Polymerization initiator V-601 (post addition) | 2.2 | 0.3 | - | - |
| | Reaction | Polymerization time after dripping V-601 | 3 | 6 | 12 | 24 |
| | | Polymerization time after adding V-601 | 9 | 6 | - | - |
| | Analysis | GPC peak top molecular weight | 15,900 | 17,200 | 17,000 | 18,000 |
| | | Residual A-1 (ppm) | 1,330 | 2,350 | 5,200 | 2,740 |

### [Example 3]

163 g of IPA was inserted into a 1 liter four-neck flask equipped with a stirring device, a thermometer, and a reflux tube. The mixture was bubbled with nitrogen gas, and then sufficiently degassed and heated to 70°C. 110 g (55% by weight) of methyl methacrylate, 10 g (5% by weight) of n-butyl acrylate, 80 g (40% by weight) of the carbosiloxane dendrimer monomer (A-1), 4.4 g (2.2% by weight) of 1,1,3,3-tetramethylbutylperoxy-2-ethylhexanoate (PEROCTA O manufactured by NOF Corporation), and 107 g of IPA were introduced and dissolved in a dripping funnel. In a nitrogen atmosphere, the monomer mixture was dripped through a dripping funnel over 1 hour while being maintained at 70°C. After the dripping was completed, heating and stirring were performed for 4 hours under a nitrogen atmosphere, and then a solution of 4.4 g (2.2% by weight) of PEROCTA (registered trademark) O (1,1,3,3-tetramethylbutylperoxy-2-ethylhexanoate) dissolved in 30 g of IPA was added. After further stirring for 4 hours, a reaction product was obtained. Analysis by GPC revealed that the polymer had a peak top molecular weight of 19,400. Furthermore, analysis by high-performance liquid chromatography revealed that 1,350 ppm of carbosiloxane dendrimer monomer (A-1) remained.

### [Example 4]

163 g of IPA was inserted into a 1 liter four-neck flask equipped with a stirring device, a thermometer, and a reflux tube. The mixture was bubbled with nitrogen gas, and then sufficiently degassed and heated to 70°C. 110 g (55% by weight) of methyl methacrylate, 10 g (5% by weight) of n-butyl acrylate, 80 g (40% by weight) of the carbosiloxane dendrimer monomer (A-1), 4.4 g (2.2% by weight) of PEROCTA O, and 107 g of IPA were introduced and dissolved in a dripping funnel. In a nitrogen atmosphere, the monomer mixture was dripped through a dripping funnel over 1 hour while being maintained at 70°C. After the dripping was completed, heating and stirring were performed for 6 hours under a nitrogen atmosphere, and then a solution of 0.6 g (0.3% by weight) of PEROCTA (registered trademark) O (1,1,3,3-tetramethylbutylperoxy-2-ethylhexanoate) dissolved in 30 g of IPA was added. After further stirring for 6 hours, a reaction product was obtained. Analysis by GPC revealed that the polymer had a peak top molecular weight of 18,700. Furthermore, analysis by high-performance liquid chromatography revealed that 1,320 ppm of carbosiloxane dendrimer monomer (A-1) remained.

### [Comparative Example 3]

163 g of IPA was inserted into a 1 liter four-neck flask equipped with a stirring device, a thermometer, and a reflux tube. The mixture was bubbled with nitrogen gas, and then sufficiently degassed and heated to 70°C. 110 g (55% by weight) of methyl methacrylate, 10 g (5% by weight) of n-butyl acrylate, 80 g (40% by weight) of the carbosiloxane dendrimer monomer (A-1), 4.4 g (2.2% by weight) of PEROCTA O, and 107 g of IPA were introduced and dissolved in a dripping funnel. In a nitrogen atmosphere, the monomer mixture was dripped through a dripping funnel over 1 hour while being maintained at 70°C. After dripping was completed, heating and stirring were performed for 12 hours under a nitrogen atmosphere to obtain a reaction product. Analysis by GPC revealed that the polymer had a peak top molecular weight of 17,000. Furthermore, analysis by high-performance liquid chromatography revealed that 9,580 ppm of carbosiloxane dendrimer monomer (A-1) remained.

### [Summary 3]

The aforementioned results are summarized in Table 3. The same trends and results were obtained using a peroxide type initiator as the polymerization initiator.

**[Table 3]**

| | | | Examples | | Comparative Examples |
|---|---|---|---|---|---|
| | | | 3 | 4 | 3 |
| Copolymer | Composition | Methyl methacrylate | 55 | 55 | 55 |
| | | N-butyl acrylate | 5 | 5 | 5 |
| | | A-1 | 40 | 40 | 40 |
| | | Initiator PEROCTA O | 2.2 | 2.2 | 2.2 |
| | | Initiator PEROCTA O (post-addition) | 2.2 | 0.3 | - |
| | Reaction | Polymerization time after dripping PEROCTA O | 4 | 6 | 12 |
| | | Polymerization time after adding PEROCTA O | 4 | 6 | - |
| | Analysis | GPC peak top molecular weight | 19,400 | 18,700 | 17,000 |
| | | Residual A-1 (ppm) | 1,350 | 1,320 | 9,580 |

In the following Example 5, Example 6, Comparative Example 4 and Comparative Example 5, studies were conducted with polymers having different monomer compositions.

### [Example 5]

163 g of IPA was inserted into a 1 liter four-neck flask equipped with a stirring device, a thermometer, and a reflux tube. The mixture was bubbled with nitrogen gas, and then sufficiently degassed and heated to 75°C. 76 g (38% by weight) of methyl methacrylate, 24 g (12% by weight) of n-butyl acrylate, 100 g (50% by weight) of the carbosiloxane dendrimer monomer (A-1), 2 g (1% by weight) of V-601, and 107 g of IPA were introduced and dissolved in a dripping funnel. In a nitrogen atmosphere, the monomer mixture was dripped through a dripping funnel over 3 hours while being maintained at 75°C. After dripping was completed, heating and stirring were performed for 4 hours under a nitrogen atmosphere, and then n-butyl acrylate 0.6 g (0.3% by weight) and a solution of 8 g (4% by weight) of V-601 dissolved in 30 g of IPA were added. After further stirring for 6 hours, a reaction product with a nonvolatile content of 40.0% was obtained. Analysis by GPC revealed that the polymer had a peak top molecular weight of 23,100. Furthermore, analysis by high-performance liquid chromatography revealed that 1,380 ppm of carbosiloxane dendrimer monomer (A-1) remained.

### [Example 6]

163 g of IPA was inserted into a 1 liter four-neck flask equipped with a stirring device, a thermometer, and a reflux tube. The mixture was bubbled with nitrogen gas, and then sufficiently degassed and heated to 70°C. 76 g (38% by weight) of methyl methacrylate, 24 g (12% by weight) of n-butyl acrylate, 100 g (50% by weight) of the carbosiloxane dendrimer monomer (A-1), 2 g (1% by weight) of V-601, and 107 g of IPA were introduced and dissolved in a dripping funnel. In a nitrogen atmosphere, the monomer mixture was dripped through a dripping funnel over 3 hours while being maintained at 70°C. After dripping was completed, heating and stirring were performed for 4 hours under a nitrogen atmosphere, and then n-butyl acrylate 0.6 g (0.3% by weight) and a solution of 8 g (4% by weight) of V-601 dissolved in 30 g of IPA were added. After further stirring for 2 hours, a reaction product with a nonvolatile content of 39.8% was obtained. Analysis by GPC revealed that the polymer had a peak top molecular weight of 28,900. Furthermore, analysis by high-performance liquid chromatography revealed that 2,240 ppm of carbosiloxane dendrimer monomer (A-1) remained.

### [Comparative Example 4]

163 g of IPA was inserted into a 1 liter four-neck flask equipped with a stirring device, a thermometer, and a reflux tube. The mixture was bubbled with nitrogen gas, and then sufficiently degassed and heated to 70°C. 76 g (38% by weight) of methyl methacrylate, 24 g (12% by weight) of n-butyl acrylate, 100 g (50% by weight) of the carbosiloxane dendrimer monomer (A-1), 2 g (1% by weight) of V-601, and 107 g of IPA were introduced and dissolved in a dripping funnel. In a nitrogen atmosphere, the monomer mixture was dripped through a dripping funnel over 3 hours while being maintained at 70°C. After dripping was completed, heating and stirring were performed for 1 hour under a nitrogen atmosphere, and then a solution of 2 g (1% by weight) of V-601 dissolved in 30 g of IPA was added. After further stirring for 2 hours, a reaction product with a nonvolatile content of 40.7 mass% was obtained. Analysis by GPC revealed that the polymer had a peak top molecular weight of 28,400. Furthermore, analysis by high-performance liquid chromatography revealed that 4,320 ppm of carbosiloxane dendrimer monomer (A-1) remained.

### [Comparative Example 5]

163 g of IPA was inserted into a 1 liter four-neck flask equipped with a stirring device, a thermometer, and a reflux tube. The mixture was bubbled with nitrogen gas, and then sufficiently degassed and heated to 70°C. 76 g (38% by weight) of methyl methacrylate, 24 g (12% by weight) of n-butyl acrylate, 100 g (50% by weight) of the carbosiloxane dendrimer monomer (A-1), 2 g (1% by weight) of V-601, and 107 g of IPA were introduced and dissolved in a dripping funnel. In a nitrogen atmosphere, the monomer mixture was dripped through a dripping funnel over 3 hours while being maintained at 70°C. After dripping was completed, heating and stirring were performed for 7 hours under a nitrogen atmosphere to obtain a reaction product with a nonvolatile content of 40.9 mass%. Analysis by GPC revealed that the polymer had a peak top molecular weight of 29,300. Furthermore, analysis by high-performance liquid chromatography revealed that 14,750 ppm of carbosiloxane dendrimer monomer (A-1) remained.

### [Summary 4]

The aforementioned results are summarized in Table 4. In the polymerization of monomers with different compositions, the residual amount of carbosiloxane dendrimer monomers (A-1) with inferior reactivity could be further reduced by adding a low-molecular monomer together with the polymerization initiator.

**[Table 4]**

| | | | Examples | | Comparative Examples | |
|---|---|---|---|---|---|---|
| | | | 5 | 6 | 4 | 5 |
| Copolymer | Composition | Methyl methacrylate | 38 | 38 | 38 | 38 |
| | | N-butyl acrylate | 12 | 12 | 12 | 12 |
| | | A-1 | 50 | 50 | 50 | 50 |
| | | Polymerization initiator V-601 | 1 | 1 | 1 | 1 |
| | | Polymerization initiator V-601 (post addition) | 4 | 4 | 1 | - |
| | | N-butyl acrylate (post addition) | 0.3 | 0.3 | - | - |
| | Reaction | Polymerization time after dripping V-601 | 4 | 4 | 1 | 7 |
| | | Polymerization time after adding V-601 | 6 | 2 | 8 | - |
| | Analysis | GPC peak top molecular weight | 23,100 | 28,900 | 28,400 | 29,300 |
| | | Residual A-1 (ppm) | 1,380 | 2,240 | 4,320 | 14,750 |

### [Examples 7 to 11]

Hereinafter, for the polymers obtained from the examples described above, the reaction solvent was replaced by an oil agent (such as isododecane or the like) to obtain a liquid copolymer composition. The details and properties thereof are summarized in Table 5. Note that the nonvolatile content is the vinyl copolymer having a carbosiloxane dendrimer structure in each example, and the amount of component (A-1) in these liquid copolymer compositions is reduced to 1000 ppm or less.

### [Example 7]

120 g of isododecane (MARUKASOL R, manufactured by Maruzen Petrochemical) was added in 200 g of an isopropyl alcohol solution of the vinyl-based polymer of Example 1, and then IPA was distilled off at 120°C. Under reduced pressure, unreacted monomers were distilled off to obtain a liquid composition. The nonvolatile content was 37.0 mass% and the kinematic viscosity was 27 mm2/s. The contact angle (water) was 105° and the contact angle (artificial sebum) was 52°. The film was not sticky and was useful as a film agent for cosmetic materials.

### [Example 8]

120 g of MARUKASOL R was added to 200 g of the isopropyl alcohol solution of the vinyl-based polymer of Example 2, and IPA was distilled off at 120°C. Under reduced pressure, unreacted monomers were distilled off to obtain a liquid composition. The nonvolatile content was 40.5 mass% and the kinematic viscosity was 66 mm2/s.

### [Example 9]

120 g of MARUKASOL R was added to 200 g of the isopropyl alcohol solution of the vinyl-based polymer of Example 3, and IPA was distilled off at 120°C. Under reduced pressure, unreacted monomers were distilled off to obtain a liquid composition. The nonvolatile content was 41.6 mass% and the kinematic viscosity was 172 mm2/s.

### [Example 10]

120 g of dodecane (PARAFOL 12-97, manufactured by Sasol) was added in 200 g of an isopropyl alcohol solution of the vinyl-based polymer of Example 5, and then IPA was distilled off at 120°C. Under reduced pressure, unreacted monomers were distilled off to obtain a liquid composition. The nonvolatile content was 39.4 mass% and the kinematic viscosity was 76 mm2/s. The contact angle (water) was 113° and the contact angle (artificial sebum) was 59°. The film was not sticky and was useful as a film agent for cosmetic materials.

### [Example 11]

120 g of a mixture of undecane and tridecane (CETIOL ULTIMATE, manufactured by BASF) was added to 200 g of the isopropyl alcohol solution of the vinyl-based polymer of Example 5, and IPA was distilled off at 120°C. Under reduced pressure, unreacted monomers were distilled off to obtain a liquid composition. The nonvolatile content was 37.6 mass% and the kinematic viscosity was 49 mm2/s. The contact angle (water) was 109° and the contact angle (artificial sebum) was 51°. The film was not sticky and was useful as a film agent for cosmetic materials.

**[Table 5]**

| | | | Examples | | | | |
|---|---|---|---|---|---|---|---|
| | | | 7 | 8 | 9 | 10 | 11 |
| Liquid composition | Composition | Copolymer IPA solution obtained in Example 1 | 100 | | | | |
| | | Copolymer IPA solution obtained in Example 2 | | 100 | | | |
| | | Copolymer IPA solution obtained in Example 3 | | | 100 | | |
| | | Copolymer IPA solution obtained in Example 5 | | | | 100 | 100 |
| | | Isododecane | 60 | 60 | 60 | | |
| | | Dodecane | | | | 60 | |
| | | Undecane/tridecane | | | | | 60 |
| | Analysis | Non-volatile content (mass%) | 37.0 | 40.5 | 41.6 | 39.4 | 37.6 |
| | | Kinematic viscosity (mm2/s) | 27 | 66 | 172 | 76 | 49 |

## Claims

1. A copolymer of
(a1) an unsaturated monomer having a carbosiloxane dendrimer structure having a radically polymerizable organic group, and
(a2) an unsaturated monomer having a radically polymerizable vinyl group, which is different from component (a1), wherein
the amount of unreacted unsaturated monomers (a1) and saturated monomers derived from the unreacted unsaturated monomers (a1) is 2500 ppm or less relative to the copolymer.

2. The copolymer according to claim 1, wherein the mass% of the aforementioned component (a1) is 20 mass% or more relative to the total mass of components (a1) and (a2) configuring the copolymer.

3. The copolymer according to claim 1 or 2, wherein component (a1) is an unsaturated monomer having a carbosiloxane dendrimer structure as expressed by formula (1). Formula (1): 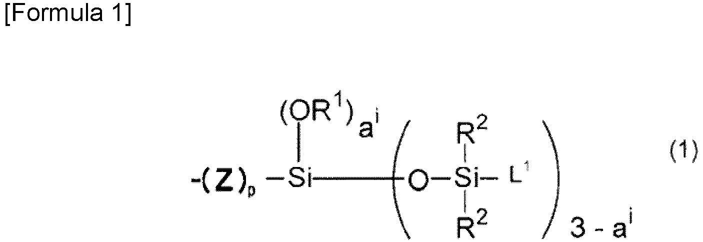 {where
Z represents a divalent organic group;
P represents 0 or 1;
R¹ and R² independently represent an alkyl group having 1 to 10 carbon atoms, an aryl group, or an aralkyl group; and
L¹ represents a silyl alkyl group as expressed by the following Formula (2) when i = 1 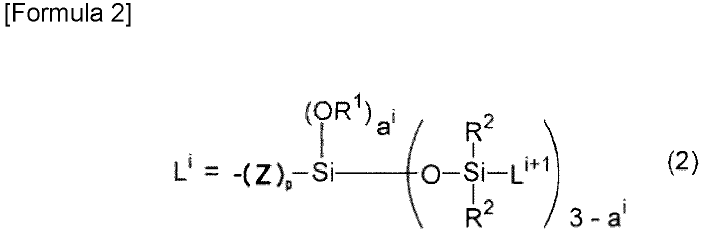 (where
Z and p are the same as described above;
R¹ and R² are the same as described above;
i represents an integer from 1 to 10 indicating the total hierarchical number of silyl alkyl groups; and
Lⁱ⁺¹ represents a group selected from a group consisting of a hydrogen atom, alkyl groups having 1 to 10 carbon atoms, aryl groups, aralkyl groups, and the silylalkyl groups; however, when i = c (c represents an integer from 1 to 10 indicating the hierarchy of the silylalkyl group), Lⁱ⁺¹ represents a hydrogen atom, an alkyl group having 1 to 10 carbon atoms, an aryl group, or an aralkyl group, but represents the silylalkyl group when i < c, and aⁱ represents an integer from 0 to 3)}.

4. A copolymer composition, comprising:
the copolymer according to any one of claims 1 to 3; and
one or more types of (C) alcohols and/or (D) oil agents.

5. A cosmetic raw material, comprising the copolymer according to any one of claims 1 to 3.

6. A film-forming agent, comprising the copolymer according to any one of claims 1 to 3.

7. A cosmetic material, comprising the copolymer according to any one of claims 1 to 3.

8. A cosmetic material, comprising the copolymer composition according to claim 4.

9. A cosmetic material, comprising:
the copolymer according to any one of claims 1 to 3; and
at least one type selected from a group consisting of (B) water, (C) alcohols, (D) oil agents, (E) powders or coloring agents, (F) surfactants, (G) oil-soluble gelling agents, (H) organically modified clay minerals, (I) silicone resins, (J) silicone gums, (K) silicone elastomers, (L) organically modified silicones, (M) ultraviolet light blocking components, and (N) water-soluble polymers.

10. The cosmetic material according to any one of claims 7 to 9, which is one or more types of cosmetic material selected from skin care products, hair products, antiperspirant products, deodorant products, makeup products, and ultraviolet light blocking products.

11. A method of manufacturing the copolymer according to any one of claims 1 to 3, comprising:
a step of adding a polymerization initiator to
(a1) the unsaturated monomer having a carbosiloxane dendrimer structure having a radically polymerizable organic group, and
(a2) the unsaturated monomer having a radically polymerizable vinyl group, which is different from component (a1)
to perform a radical polymerization reaction; and
step (I): a step (Ia) of further adding the same or different polymerization initiator than the initially added polymerization initiator at least two hours after adding the initial polymerization initiator for the radical polymerization reaction.

12. The method of manufacturing the copolymer according to claim 11, wherein the aforementioned step (I) further includes a step (Ib) of adding a monomer (a2') having a radically polymerizable vinyl group with a boiling point less than 160°C at ambient pressure (1 atm) to advance the radical polymerization reaction of unreacted component (a1).

13. The method of manufacturing the copolymer according to claim 11 or claim 12, further comprising:
step (II): a step of distilling off unreacted unsaturated monomers or saturated monomers remaining in the system at ambient pressure or under reduced pressure after step (I).

14. The method of manufacturing the copolymer according to any one of claims 11 to 13, further comprising:
step (III): a step of performing a hydrogenation reaction on an unreacted unsaturated monomer remaining in the system to convert the unsaturated monomer to a saturated monomer.

15. The method of manufacturing the copolymer according to any one of claims 11 to 14, further comprising:
step (IV): a step of adding one or more types of (C) alcohols and/or (D) oil agents in the system and removing a reaction solvent for a radical polymerization reaction.
